# EUROPÄISCHE PATENTSCHRIFT

(11) **EP 4 063 007 B1**
(45) Veröffentlichungstag und Bekanntmachung des Hinweises auf die Patenterteilung: **09.08.2023**
(21) Anmeldenummer: 21164046.1
(22) Anmeldetag: 22.03.2021
(51) Int. Cl.: B01F 33/501, B01F 35/71, B01F 35/75, A61B 17/88, B01F 101/20

(54) **VERFAHREN UND VORRICHTUNG ZUM MISCHEN VON KNOCHENZEMENT MIT DRUCKENTLASTUNG**
METHOD AND DEVICE FOR MIXING BONE CEMENT WITH PRESSURE RELIEF
PROCÉDÉ ET DISPOSITIF DE MÉLANGE DU CIMENT OSSEUX À DÉPRESSURISATION

(43) Veröffentlichungstag der Anmeldung: 28.09.2022
(73) Patentinhaber: Heraeus Medical GmbH, 61273 Wehrheim (DE)
(72) Erfinder: Vogt, Sebastian Dr., 61273 Wehrheim (DE); Kluge, Thomas Dr., 61273 Wehrheim (DE)
(74) Vertreter: Schultheiss & Sterzel Patentanwälte PartG mbB

(56) Entgegenhaltungen:
- EP-A1- 3 505 237
- WO-A1-97/18031
- DE-A1-102017 113 126
- US-A- 4 613 326
- US-A1- 2012 155 214

## Beschreibung

Die Erfindung betrifft ein Verfahren zum Herstellen eines Knochenzements aus einer Monomerflüssigkeit und einem Zementpulver als Ausgangskomponenten des Knochenzements.

Die Erfindung betrifft auch eine Vorrichtung zur Herstellung eines Knochenzements, insbesondere eines pastenförmigen Polymethylmethacrylat-Knochenzements, aus einer Monomerflüssigkeit und einem Zementpulver.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Die Monomerkomponente enthält im Allgemeinen das Monomer Methylmethacrylat und einen darin gelösten Aktivator (N,N-Dimethyl-p-toluidin). Die Pulverkomponente, vorliegend als Zementpulver oder auch als Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt.

PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2, der EP 0 796 653 A2, der US 5 588 745 A, der US 2018/333 176 A1, der US 2018/310 974 A1, der US 2018/289 406 A1, der US 2018/132 919 A1, der US 2018/132 917 A1 und der US 2018/256 233 A1 vorgeschlagen.

Das Patent DE 10 2009 031 178 B3 offenbart eine Lager- und Mischvorrichtung als Full-Prepacked-Mischsystem, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können.

Polymethylmethacrylat-Knochenzemente werden nach Vermischung des Zementpulvers mit der flüssigen Monomerkomponente im noch nicht ausgehärteten, pastenförmigen Zustand als Knochenzementteig angewendet. Bei Verwendung von Mischvorrichtungen befindet sich der Knochenzementteig im Fall von Pulver-Flüssigkeits-Zementen in einer Kartusche. Bei der Herstellung solcher konventioneller PMMA-Knochenzemente, wird nach der Vermischung der beiden Ausgangskomponenten der gebildete Knochenzementteig mit Hilfe von manuell bedienbaren Auspressvorrichtungen ausgepresst. Aus der Kartusche wird der Knochenzementteig durch Bewegung eines Austragskolbens herausgedrückt.

Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar.

In den Patenten EP 3 320 869 B1 und EP 3 320 870 B1 wird ein Zementiersystem beschrieben, bei denen die Vermischung der Monomerflüssigkeit mit dem Zementpulver durch Einpressen von Monomerflüssigkeit in verdichtetes Zementpulver erfolgt. Es zeigte sich in eigenen praktischen Versuchen, dass mit dieser Vorrichtung zuverlässig Polymethylmethacrylat-Knochenzementteig hergestellt werden kann. Mitunter kann es durch Schwankungen des Monomerflüssigkeitsvolumens im Monomerflüssigkeitsbehälter zu unerwünschten Einspritzungen kleinster Monomerflüssigkeitsvolumina in den gequollenen Zementteig während des Auspressens des gebildeten Knochenzements kommen. Diese Einspritzungen sind im Volumenbereich von wenigen Mikrolitern.

Die EP 3 505 237 A1 offenbart ein Verfahren entsprechend dem Oberbegriff des Anspruchs 1 und eine Vorrichtung entsprechend dem Oberbegriff des Anspruchs 9.

Die Aufgabe der vorliegenden Erfindung besteht darin, die Nachteile des Stands der Technik zu überwinden. Insbesondere besteht die Aufgabe der Erfindung in der Entwicklung eines Verfahrens und einer Vorrichtung bei denen ein Nachpressen von Monomerflüssigkeit nach der Benetzung des Zementpulvers mit der Monomerflüssigkeit vermieden werden kann. Die Vorrichtung soll möglichst einfach bedienbar und kostengünstig zu fertigen sein. Das Verfahren soll einfach anwendbar sein und kostengünstig umsetzbar sein. Die Vorrichtung soll insbesondere zur Umsetzung des Verfahrens geeignet und vorgesehen sein.

Die Aufgabe der Erfindung besteht somit in der Entwicklung eines Verfahrens und einer Vorrichtung zur Herstellung von Knochenzement, insbesondere zum Vermischen und Austragen von Polymethylmethacrylat-Knochenzement, basierend auf den Lehren der Patentschriften EP 3 320 869 B1 und EP 3 320 870 B1, das einfach und kostengünstig realisierbar ist und mit dem gleichmäßig ein möglichst homogener Knochenzement herstellbar sein soll. Das zu entwickelnde Verfahren soll so gestaltet werden, dass ein unerwünschtes Einspritzen von kleinsten Monomerflüssigkeitsvolumina in den gebildeten Zementteig während des Auspressvorgangs sicher verhindert wird. Weiterhin sollen auch die Zementiervorrichtung und das Verfahren so weiterentwickelt werden, dass ein Einspritzen auch kleiner und kleinster Monomerflüssigkeitsvolumina in den Knochenzement während des Austragens des gebildeten Knochenzements sicher vermieden werden können.

Die Aufgaben der Erfindung werden gelöst durch ein Verfahren zur Herstellung eines Knochenzements, insbesondere eines pastenförmigen Polymethylmethacrylat-Knochenzements, wobei der Knochenzement aus einem Zementpulver und einer Monomerflüssigkeit mit einer Vorrichtung zum Mischen des Knochenzements hergestellt wird, die Vorrichtung aufweisend
a) eine Kartusche mit einem zylindrischen Innenraum,
b) ein Zementpulver zur Herstellung des Knochenzements,
c) eine Monomerflüssigkeit zur Herstellung des Knochenzements, wobei die Monomerflüssigkeit in einem Monomerflüssigkeitsbehälter enthalten ist,
d) einen Kartuschenkopf mit einer Austragsöffnung zum Austreiben des Knochenzements, wobei der Kartuschenkopf den zylindrischen Innenraum der Kartusche an einer Vorderseite der Kartusche bis auf die Austragsöffnung verschließt,
e) einen Verschluss, wobei der Verschluss für Gase durchlässig und für Pulverpartikel des Zementpulvers undurchlässig ist und wobei der Verschluss in der Austragsöffnung angeordnet ist und aus der Austragsöffnung entfernbar ist,
f) einen Antriebskolben, wobei der Antriebskolben für Gase und die Monomerflüssigkeit undurchlässig ist, wobei der Antriebskolben in Richtung des Kartuschenkopfs beweglich im zylindrischen Innenraum der Kartusche angeordnet ist,
g) einen Mittelkolben, wobei der Mittelkolben für Gase und die Monomerflüssigkeit durchlässig und für die Pulverpartikel des Zementpulvers undurchlässig ist, wobei der Mittelkolben in Richtung des Kartuschenkopfs beweglich im zylindrischen Innenraum der Kartusche angeordnet ist und zwischen dem Antriebskolben und dem Kartuschenkopf angeordnet ist, wobei der Mittelkolben den zylindrischen Innenraum der Kartusche in einen ersten Hohlraum und einen zweiten Hohlraum trennt, wobei der erste Hohlraum an einer Vorderseite durch den Kartuschenkopf und den Verschluss, gegenüberliegend durch den Mittelkolben und seitlich durch eine Innenwand der Kartusche begrenzt ist und wobei der zweite Hohlraum an einer Vorderseite durch den Mittelkolben, gegenüberliegend durch den Antriebskolben und seitlich durch die Innenwand der Kartusche begrenzt ist, wobei das Zementpulver in dem ersten Hohlraum angeordnet ist und wobei der Monomerflüssigkeitsbehälter in dem zweiten Hohlraum angeordnet ist,
wobei das Verfahren die folgenden Schritte aufweist:
A) Bewegen des Antriebskolbens in Richtung des Kartuschenkopfs,
B) Öffnen des Monomerflüssigkeitsbehälters oder Zerbrechen des Monomerflüssigkeitsbehälters durch die Bewegung des Antriebskolbens in Richtung Kartuschenkopf und dadurch Freisetzen der Monomerflüssigkeit in dem zweiten Hohlraum,
C) Auspressen überstehender Gase durch den Mittelkolben, durch das Zementpulver und durch den Verschluss hindurch in die Umgebung der Vorrichtung mittels der Bewegung des Antriebskolbens,
D) Einpressen der Monomerflüssigkeit durch den Mittelkolben in das Zementpulver durch weiteres Bewegen des Antriebskolbens in Richtung Kartuschenkopf,
E) Verdrängen von Gasen zwischen den Pulverpartikeln mit der einfließenden Monomerflüssigkeit, wobei die Gase durch den Verschluss in die Umgebung der Vorrichtung entweichen,
F) Benetzen der Pulverpartikel des Zementpulvers und
G) Druckentlasten des zweiten Hohlraums nach den Schritten A) bis F), wobei das Druckentlasten des zweiten Hohlraums durch ein teilweises Entfernen der in dem zweiten Hohlraum enthaltenen Monomerflüssigkeit erfolgt.

Vorzugsweise ist der Verschluss für Gase und für die Monomerflüssigkeit durchlässig.

Der zylindrische Innenraum der Kartusche hat eine zylindrische Geometrie, gegebenenfalls bis auf die von einer Druckentlastungvorrichtung oder der Verbindung zur Druckentlastungsvorrichtung verursachten Asymmetrien, wie beispielsweise durch zumindest eine Nut in einer Innenwand der Kartusche oder durch mindestens eine Verbindung in der Kartusche zu einem Reservoir für die Monomerflüssigkeit. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Kartusche kann also durch den Zylindermantel eines Zylinders mit beliebiger Grundfläche realisiert sein, insbesondere mit unterschiedlicher Grundfläche realisiert sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche für den zylindrischen Innenraum der Kartusche bevorzugt, da diese am einfachsten zu fertigen ist.

Es kann erfindungsgemäß vorgesehen sein, dass das Zementpulver in dem ersten Hohlraum verdichtet ist.

Der Antriebskolben ist vorzugsweise entlang der Zylinderachse des zylindrischen Innenraums beweglich im zylindrischen Innenraum der Kartusche angeordnet.

Der Mittelkolben ist vorzugsweise entlang der Zylinderachse des zylindrischen Innenraums beweglich im zylindrischen Innenraum der Kartusche zwischen dem Antriebskolben und dem Kartuschenkopf angeordnet.

Für Pulverpartikel undurchlässig bedeutet, dass zumindest ein Großteil der Masse der Pulverpartikel, aus dem das Zementpulver besteht, nicht hindurchrieseln oder hindurchfallen kann. Bevorzugt können zumindest 90% der Masse der Pulverpartikel, aus dem das Zementpulver besteht, nicht hindurchrieseln oder hindurchfallen. Besonders bevorzugt können zumindest 99% der Masse der Pulverpartikel, aus dem das Zementpulver besteht, nicht hindurchrieseln oder hindurchfallen. Ganz besonders bevorzugt können zumindest 99,9% der Masse der Pulverpartikel, aus dem das Zementpulver besteht, nicht hindurchrieseln oder hindurchfallen. Die prozentualen Anteile beziehen sich dabei auf die Gesamtmasse des Zementpulvers und nicht auf die Gesamtanzahl der Pulverpartikel des Zementpulvers.

Das Druckentlasten kann mit Hilfe einer eigens dafür vorgesehenen Druckentlastungsvorrichtung geschehen, was auch erfindungsgemäß bevorzugt ist. Alternativ kann die Druckentlastung aber auch beispielsweise durch ein Verformen des Antriebskolbens oder des Mittelkolbens oder durch ein Zurückziehen oder Zurückweichen des Antriebskolbens erfolgen oder durch ein Aufsaugen eines Teils der Monomerflüssigkeit mit einem saugfähigen Material, wie beispielsweise einem schwammartigen Stoff, wie einer Zellulose oder wie einem Zellstoff.

Es kann vorgesehen sein, dass der Druck in dem zweiten Hohlraum durch die Druckentlastung zumindest um 30% reduziert wird, besonders bevorzugt um zumindest 60% reduziert wird, ganz besonders bevorzugt um zumindest 90% reduziert wird.

Hierdurch wird sichergestellt, dass im zweiten Hohlraum eine Druckreduzierung erreicht wird, die ein Nachpressen von Monomerflüssigkeit aus dem zweiten Hohlraum in den ersten Hohlraum deutlich reduziert oder weitgehend verhindert. Dem Fachmann sind Verfahren zur Bestimmung des absoluten Drucks in dem zweiten Hohlraum bekannt. Der Druck wird mit dem Rest der Monomerflüssigkeit in dem zweiten Hohlraum als hydrostatischer Druck vermittelt und kann als solcher auf übliche und dem Fachmann bekannter Weise gemessen werden. Hierzu kann beispielsweise zu Versuchszwecken ein elektronischer Drucksensor auf der Innenwand des zweiten Hohlraums (im komprimierten Zustand) angebracht sein beziehungsweise werden, der durch eine Bohrung in der Kartuschenwandung der Kartusche elektrisch kontaktiert wird.

Des Weiteren kann vorgesehen sein, dass das Bewegen des Antriebskolbens in Richtung des Kartuschenkopfs durch eine äußere Auspressvorrichtung angetrieben wird, wobei bevorzugt die Vorrichtung zum Mischen des Knochenzements in die Auspressvorrichtung eingesetzt wird und/oder an der Auspressvorrichtung befestigt wird.

Hierdurch können die Antriebskolben mehrerer Vorrichtungen zum Mischen des Knochenzements nacheinander wirksam mit der gleichen Auspressvorrichtung angetrieben werden. Die Auspressvorrichtung kann rein mechanisch, motorgetrieben oder auch mit einem Druckgas angetrieben werden.

Ferner kann vorgesehen sein, dass in Schritt F) die Pulverpartikel des Zementpulvers vollständig von der Monomerflüssigkeit benetzt werden.

Hierdurch wird sichergestellt, dass ein homogener Knochenzement erzeugt wird und dass die Druckentlastung in Schritt G) erst dann erfolgt, wenn das Zementpulver vollständig mit der Monomerflüssigkeit benetzt ist.

Des Weiteren kann vorgesehen sein, dass das Verfahren die folgenden chronologischen Schritte aufweist:
H) Entfernen des Verschlusses aus dem Kartuschenkopf nach Schritt G) und
I) Auspressen des Knochenzements aus dem ersten Hohlraum und durch die Austragsöffnung durch eine Bewegung des Mittelkolbens in Richtung des Kartuschenkopfs, wobei der Mittelkolben von dem Antriebskolben in Richtung des Kartuschenkopfs gedrückt wird.

Durch diese Schritte wird der Knochenzement für die weitere Anwendung bereitgestellt. Beispielsweise kann der Knochenzement im Anschluss an Schritt I) für eine medizinische Anwendung verwendet werden. Ebenfalls beispielsweise kann der Knochenzement in einem Gefäß aufgefangen werden, aus dem heraus danach eine medizinische Anwendung erfolgen kann. Der Schritt I) selbst erlaubt hingegen noch keine medizinische Anwendung, da keine Kontaktierung des Knochenzements mit einem menschlichen oder tierischen Körper erfolgt.

Im Anschluss an das Auspressen gemäß Schritt I) kann der Knochenzementteig appliziert werden. Der Knochenzement kann in eine Auffangschale gedrückt werden oder auf ein Implantat vor dem Implantieren des Implantats aufgetragen werden.

Es kann insbesondere auch vorgesehen sein, dass das Verfahren nicht zur medizinischen, diagnostischen oder therapeutischen Behandlung eines menschlichen oder tierischen Körpers angewendet wird.

Hierdurch wird klargestellt, dass es sich bei dem Verfahren nicht um ein medizinisches Verfahren handelt, insbesondere nicht um ein von der Patentierung ausgeschlossenes medizinisches Verfahren handelt.

Ferner kann vorgesehen sein, dass das Druckentlasten in Schritt G) durch eine manuelle oder eine automatische Betätigung einer Druckentlastungsvorrichtung der Vorrichtung zum Mischen des Knochenzements erfolgt, wobei bevorzugt die automatische Betätigung der Druckentlastungsvorrichtung durch eine Bewegung des Antriebskolbens ausgelöst wird und/oder durch eine Bewegung des Verschlusses aus dem Kartuschenkopf ausgelöst wird und besonders bevorzugt durch die Bewegung des Verschlusses aus dem Kartuschenkopf angetrieben wird.

Hierdurch wird die Durchführung des Verfahrens und die Anwendung der Vorrichtung vereinfacht. Bevorzugt wird die Bewegung des Verschlusses von dem Knochenzement angetrieben, sobald dieser aufgrund der Benetzung mit Monomerflüssigkeit fließfähig geworden ist.

Es kann also vorgesehen sein, dass das Druckentlasten beziehungsweise eine selbsttätige Druckentlastungsvorrichtung durch die Bewegung des Verschlusses aus dem Kartuschenkopf angetrieben und ausgelöst wird. Der Verschluss bewegt sich erst aus dem Kartuschenkopf, wenn das gesamte Zementpulver mit der Monomerflüssigkeit benetzt ist. Die Verblockung der unbenetzten Pulverpartikel des Zementpulvers untereinander verhindert zuvor eine axiale Bewegung des Verschlusses. Dann kann der Antriebskolben bei seiner Bewegung in Richtung Kartuschenkopf den geöffneten oder zerborstenen Monomerflüssigkeitsbehälter und den Mittelkolben in Richtung des Kartuschenkopfs bewegen. Der Verschluss kann hierzu mit einem Ventilsystem verbunden sein, das mindestens eine durchgehende Verbindung in der Kartuschenwand verschließt. Dadurch wird bei einer Bewegung des Verschlusses aus dem Kartuschenkopf das Ventilsystem geöffnet, so dass die Druckentlastung durch das Austreten der Monomerflüssigkeit aus dem zweiten Hohlraum durch die mindestens eine durchgehende Verbindung erreicht wird.

Des Weiteren kann vorgesehen sein, dass bei dem Druckentlasten in Schritt G) die Monomerflüssigkeit in ein Reservoir außerhalb der Kartusche oder in der Kartuschenwand abgeleitet wird, wobei bevorzugt das Reservoir nach außen für die Monomerflüssigkeit dicht geschlossen ist, oder bei dem Druckentlasten in Schritt G) die Monomerflüssigkeit in ein Reservoir innerhalb des Antriebskolbens, in ein Reservoir auf der dem zweiten Hohlraum gegenüberliegenden Seite des Antriebskolbens und/oder in ein Reservoir seitlich zwischen dem Antriebskolben und der Kartusche geleitet wird, wobei bevorzugt in letzterem Fall die Monomerflüssigkeit durch zumindest eine Nut in einer Innenwand der Kartusche an einem Dichtungsring des Antriebskolbens vorbei in das Reservoir seitlich zwischen dem Antriebskolben und der Kartusche geleitet wird, wenn ein vorderer Dichtungsring des Antriebskolbens über oder auf die zumindest eine Nut geschoben wird.

Es reicht aus, wenn ein kleiner Anteil (weniger als 2 ml) der Monomerflüssigkeit aus dem zweiten Hohlraum abgeleitet wird, um eine Druckentlastung im zweiten Hohlraum zu erreichen. Mit den genannten Reservoirs kann eine schnelle und unkomplizierte Ableitung der Monomerflüssigkeit aus dem zweiten Hohlraum erreicht werden. Gleichzeitig können die Reservoirs nach außen abgeschlossen sein und so eine Freisetzung der Monomerflüssigkeit in die Umgebung der Vorrichtung verhindert werden. Nach außen bedeutet in diesem Zusammenhang in die Umgebung der Vorrichtung.

Gemäß einer bevorzugten Weiterbildung kann vorgesehen sein, dass bei dem Druckentlasten in Schritt G) die Monomerflüssigkeit nicht in den ersten Hohlraum geleitet wird, bevorzugt in zumindest ein zum ersten Hohlraum separates Reservoir geleitet wird, wobei besonders bevorzugt das zumindest eine Reservoir außerhalb der Kartusche, in der Kartuschenwand, innerhalb des Antriebskolbens, auf der dem zweiten Hohlraum gegenüberliegenden Seite des Antriebskolbens und/oder seitlich zwischen dem Antriebskolben und der Kartusche angeordnet ist.

Hierdurch wird verhindert, dass Teile der Monomerflüssigkeit bei der Druckentlastung in den ersten Hohlraum gelangen und dort die Zusammensetzung des Knochenzements bereichsweise verändern. Zudem kann so eine effektive und vollständige Druckentlastung erfolgen.

Auch kann vorgesehen sein, dass bei dem Druckentlasten in Schritt G) der zweite Hohlraum mit dem geöffneten oder zerbrochenen Monomerflüssigkeitsbehälter und mit Resten der Monomerflüssigkeit gefüllt ist.

Hierdurch wird klargestellt, dass der sich im zweiten Hohlraum aufbauende hydrostatische Überdruck durch die Entfernung der Monomerflüssigkeit aus dem zweiten Hohlraum erfolgen kann.

Die der vorliegenden Erfindung zugrundeliegenden Aufgaben werden auch gelöst durch eine Vorrichtung zum Mischen eines Knochenzements, die Vorrichtung aufweisend
a) eine Kartusche mit einem zylindrischen Innenraum,
b) ein Zementpulver zur Herstellung des Knochenzements,
c) eine Monomerflüssigkeit zur Herstellung des Knochenzements, wobei die Monomerflüssigkeit in einem Monomerflüssigkeitsbehälter enthalten ist,
d) einen Kartuschenkopf mit einer Austragsöffnung zum Austreiben des Knochenzements, wobei der Kartuschenkopf den zylindrischen Innenraum der Kartusche an einer Vorderseite der Kartusche bis auf die Austragsöffnung verschließt,
e) einen Verschluss, wobei der Verschluss für Gase durchlässig und für Pulverpartikel des Zementpulvers undurchlässig ist und wobei der Verschluss in der Austragsöffnung angeordnet ist und aus der Austragsöffnung entfernbar ist,
f) einen Antriebskolben, wobei der Antriebskolben für Gase und die Monomerflüssigkeit undurchlässig ist, wobei der Antriebskolben in Richtung des Kartuschenkopfs beweglich im zylindrischen Innenraum der Kartusche angeordnet ist,
g) einen Mittelkolben, wobei der Mittelkolben für Gase und die Monomerflüssigkeit durchlässig und für die Pulverpartikel des Zementpulvers undurchlässig ist, wobei der Mittelkolben in Richtung des Kartuschenkopfs beweglich im zylindrischen Innenraum der Kartusche angeordnet ist und zwischen dem Antriebskolben und dem Kartuschenkopf angeordnet ist, wobei der Mittelkolben den zylindrischen Innenraum der Kartusche in einen ersten Hohlraum und einen zweiten Hohlraum trennt, wobei der erste Hohlraum an einer Vorderseite durch den Kartuschenkopf und den Verschluss, gegenüberliegend durch den Mittelkolben und seitlich durch eine Innenwand der Kartusche begrenzt ist und wobei der zweite Hohlraum an einer Vorderseite durch den Mittelkolben, gegenüberliegend durch den Antriebskolben und seitlich durch die Innenwand der Kartusche begrenzt ist, wobei das Zementpulver in dem ersten Hohlraum angeordnet ist und wobei der Monomerflüssigkeitsbehälter in dem zweiten Hohlraum angeordnet ist, und
h) eine Druckentlastungsvorrichtung, mit der Monomerflüssigkeit aus dem zweiten Hohlraum ableitbar oder ableitbar und aufnehmbar ist oder mit der das Volumen des zweiten Hohlraums vergrößerbar ist, wobei die Druckentlastungsvorrichtung mit dem zweiten Hohlraum verbunden oder verbindbar ist.

Eine Vergrößerung des Volumens des zweiten Hohlraums kann beispielsweise durch eine Komprimierung des Mittelkobens und/oder des Antriebskolbens erreicht werden oder durch eine Expansion der Wandung der Kartusche im Bereich des zweiten Hohlraums oder durch eine Auseinanderziehen des zweiten Hohlraums, indem beispielsweise die Kartusche zweiteilig aufgebaut ist, wobei die beiden Teile im Bereich des zweiten Hohlraums über eine Schraubverbindung miteinander verbunden sind, so dass durch eine Drehung um einige Grad eine Verlängerung des zweiten Hohlraums erfolgt.

Es kann vorgesehen sein, dass zur Aufnahme der Monomerflüssigkeit aus dem zweiten Hohlraum ein Zellstoff verwendet wird, der die Monomerflüssigkeit zumindest teilweise, vorzugsweise vollständig aufsaugt.

Es kann vorgesehen sein, dass die Druckentlastungsvorrichtung einen hohlen Stutzen aufweist, der sich aus der äußeren Oberfläche der Kartusche erstreckt, wobei bevorzugt der hohle Stutzen eine Verlängerung der mindestens einen durchgehenden Verbindung bildet oder sich über einer dünnen mit einem Dorn der Druckentlastungsvorrichtung durchstechbaren Wandung der Kartusche erstreckt.

Die Druckentlastungsvorrichtung kann eine Kappe aufweisen, die zumindest in einem nicht arretierten Zustand gegen die Kartusche, insbesondere gegen den hohlen Stutzen (sofern vorhanden), beweglich ist. Bevorzugt kann die Kappe zum Bewegen eines Dorns, einer Dichtung und/oder zum manuellen Antreiben einer Schraubbewegung verwendet werden.

Es kann vorgesehen sein, dass die Vorrichtung zum Umsetzen eines erfindungsgemäßen Verfahrens geeignet ist.

Hierdurch hat die Vorrichtung die zu dem Verfahren genannten Vorteile
Des Weiteren kann vorgesehen sein, dass die Druckentlastungsvorrichtung über mindestens eine durchgehende Verbindung in einer Wandung der Kartusche mit dem zweiten Hohlraum verbunden ist, wobei die mindestens eine durchgehende Verbindung mit mindestens einem Dichtungskörper verschlossen oder verschließbar ist, wobei bevorzugt der mindestens eine Dichtungskörper mit einer von außen manuell bedienbaren Schraube oder Schraubkappe, mit einer Stange oder mit einer Federführungsstange gegen die mindestens eine durchgehende Verbindung gedrückt ist.

Hierdurch kann eine einfach und kostengünstig aufgebaute Druckentlastungvorrichtung bereitgestellt werden, die leicht und zuverlässig von außen zu bedienen ist.

Dabei kann vorgesehen sein, dass der mindestens eine Dichtungskörper oder eine auf den mindestens einen Dichtungskörper drückende Stange oder Federführungsstange mit einer Feder von der mindestens eine durchgehende Verbindung abhebbar ist, bevorzugt die Feder die Federführungsstange, die den mindestens einen Dichtungskörper gegen die mindestens eine durchgehende Verbindung drückt, von der mindestens einen durchgehenden Verbindung weg drückt, wobei besonders bevorzugt die Feder mit einem Zapfen oder mit einem gabelförmigen Stützkörper arretiert ist und der Zapfen oder der gabelförmige Stützkörper mit dem Verschluss fest verbunden ist, so dass bei einer Bewegung des Verschlusses aus der Austragsöffnung auch der Zapfen oder der gabelförmige Stützkörper automatisch aus der Druckentlastungsvorrichtung entfernt wird und dadurch die Arretierung der Feder gelöst wird.

Hierdurch wird ein stabiler und gut zu öffnender Verschluss der mindestens einen durchgehenden Verbindung bereitgestellt. Durch die Weiterbildung kann ein automatisiertes Öffnen der mindestens einen durchgehenden Verbindung erreicht werden.

Wenn die Monomerflüssigkeit durch den Antriebskolben und durch den Mittelkolben in das verdichtete Zementpulver gepresst wird, erreicht die Monomerflüssigkeit den beweglichen Verschluss in der Austragsöffnung im Kartuschenkopf. Nur mit Monomerflüssigkeit benetztes Zementpulver kann durch Druckeinwirkung innerhalb der Kartusche verschoben werden beziehungsweise fließen. Der Verschluss wird erst dann von dem mit der Monomerflüssigkeit benetzten Zementpulver angeschoben, wenn das gesamte Zementpulver benetzt ist. Dann bewegt sich der Verschluss aus der Austragsöffnung heraus. Der Verschluss ist mit einem Stützkörper verbunden, der durch den Verschluss in Richtung Kartuschenkopf gezogen wird. Der Stützkörper blockiert die Abstützung einer Feder, die über eine Federführungsstange auf einen Dichtungskörper drückt. Wenn der Stützkörper in Richtung des Kartuschenkopfs durch den Verschluss gezogen wird, kann der Stützkörper nicht mehr die Feder abstützen. Die Feder verliert ihre Spannung und kann nicht mehr auf den Dichtungskörper drücken. Der Dichtungskörper kommt frei und gibt die zuvor verschlossene Öffnung in der Kartuschenwand frei. Die unter Druck stehende Restmenge an Monomerflüssigkeit tritt aus. Die ausgetretene Monomerflüssigkeit kann durch saugfähiges Material, wie Zellulose oder Papierscheiben aufgefangen werden. Nach dem Öffnen der mindestens einen durchgehenden Verbindung in der Kartuschenwand findet ein Druckausgleich zwischen dem zweiten Hohlraum zwischen der Oberseite des Antriebskolbens und der Unterseite des Mittelkolbens und der umgebenden Atmosphäre statt.

In einer weiteren Ausgestaltungsvariante kann die mindestens eine durchgehende Verbindung durch die Kartuschenwand durch einen Dichtungskörper reversibel verschlossen sein oder verschließbar sein, wobei der Dichtungskörper durch eine Feder über eine Federführungsstange gegen die Bohrung gepresst wird, wobei sich die Feder auf einen gabelförmigen Stützkörper stützt und der Stützkörper mit dem beweglichen Verschluss verbunden ist.

Es kann auch vorgesehen sein, dass mindestens eine durchgehende Verbindung in der Kartuschenwand angeordnet ist, die den zylindrischen Innenraum der Kartusche mit der äußeren Umgebung verbindet, wobei die mindestens eine durchgehende Verbindung reversibel verschlossen oder verschließbar ist und wobei der reversible Verschluss der mindestens einen durchgehenden Verbindung durch einen Dichtungskörper erfolgt, der durch eine Schraube oder eine Schraubkappe gegen die mindestens eine durchgehende Verbindung gepresst wird, wobei die Schraube oder die Schraubkappe manuell von außen verdrehbar ist.

Die Schraube oder die Schraubkappe ist vorzugsweise in oder an einem an einer Mantelfläche der Kartusche angeordneten hohlen Stutzen, der ein Innengewinde aufweist, eingeschraubt. Die durchgehende Verbindung in der Kartuschenwand kann so angeordnet sein, dass der Stutzen die durchgehende Verbindung umgibt. Die Schraube oder die Schraubkappe kann einen Dichtungskörper gegen die durchgehende Verbindung in der Kartuschenwand drücken.

Die durchgehende Verbindung kann dadurch druckfest verschlossen werden. Zur Druckentlastung kann die Schraube oder die Schraubkappe etwas aus ihrem Sitz herausgedreht werden. Dadurch wird der Dichtungskörper entlastet und weicht von der mindestens einen durchgehenden Verbindung zurück. Die unter Überdruck stehende Monomerflüssigkeit tritt aus. Vorteilhaft ist hinter dem Dichtungskörper in der Druckentlastungsvorrichtung ein saugfähiges Material angeordnet, das geringe Volumina an austretender Monomerflüssigkeit aufnimmt.

Der Dichtungskörper kann auch durch einen manuell zu entfernenden Keil oder mit einer Nocke auf einer manuelle drehbaren Achse gegen die durchgehende Verbindung in der Kartuschenwand gepresst werden.

Ferner kann vorgesehen sein, dass die Druckentlastungsvorrichtung einen Dorn zum Durchstechen einer Wandung der Kartusche im Bereich des zweiten Hohlraums aufweist, wobei der Dorn beweglich gegen die Kartusche gelagert ist, wobei der Dorn ein Hohldorn mit einer Kanüle ist, die mit einem Reservoir zur Aufnahme der Monomerflüssigkeit verbunden ist, oder der Dorn nach dem Durchstechen der Wandung der Kartusche manuell oder durch eine Feder zurückziehbar ist, so dass er einen mit dem Dorn gestochenen Durchgang zur Ableitung der Monomerflüssigkeit aus dem zweiten Hohlraum freilegt, wobei bevorzugt die Wandung im Bereich des Dorns dünner ist als im Rest der Kartusche.

Bei dieser Variante ist sichergestellt, dass der zweite Hohlraum vor dem Durchstechen der Wandung der Kartusche mit dem Dorn gegen die Druckentlastungsvorrichtung vollständig abgedichtet ist. Der Dorn kann erfindungsgemäß eine Furche aufweisen, durch die die Monomerflüssigkeit aus dem zweiten Hohlraum in die Druckentlastungsvorrichtung fließen kann.

Es kann erfindungsgemäß vorgesehen sein, dass zumindest ein Teil der Kartuschenwand so dünn ist, dass diese durch einen Dorn eingestochen werden kann, wobei der Dorn manuell von außen in Richtung der Kartuschenwand verschoben werden kann und die Spitze des Dorns nach dem Durchstechen der Kartuschenwand durch manuelle Kraft oder durch eine Rückstellkraft zumindest einer Feder aus dem Innenraum der Kartusche zurückgezogen wird. Nach dem Durchstechen der Kartuschenwand findet ein Druckausgleich zwischen dem zweiten Hohlraum zwischen der Oberseite des Antriebskolbens und der Unterseite des Mittelkolbens und der umgebenden Atmosphäre statt oder einem Reservoir im Inneren der Druckentlastungsvorrichtung.

Es ist auch möglich, dass die mindestens eine durchgehende Verbindung mit einer Metallmembran verschlossen ist, die durch die manuelle Bewegung des Dorns durchstochen werden kann.

Des Weiteren kann vorgesehen sein, dass der Antriebskolben mit zumindest einem Dichtungsring gegen die Innenwand Kartusche abgedichtet ist, bevorzugt mit zwei Dichtungsringen gegen die Innenwand Kartusche abgedichtet ist, wobei die zwei Dichtungsringe in einer Richtung parallel zur Zylinderachse des zylindrischen Innenraums voneinander beabstandet sind, besonders bevorzugt zumindest 5 mm voneinander beabstandet sind.

Hierdurch wird gewährleistet, dass die Monomerflüssigkeit vor der Druckentlastung aus dem zweiten Hohlraum in den ersten Hohlraum gepresst wird und nicht bereits vor der Druckentlastung in einen Zwischenraum zwischen dem Antriebskolben und der Kartusche und durch diesen Zwischenraum an dem Antriebskolben vorbei gepresst werden kann.

Auch kann vorgesehen sein, dass in einer den zweiten Hohlraum begrenzenden Innenwand der Kartusche zumindest eine Nut angeordnet ist, wobei die zumindest eine Nut parallel zur Zylinderachse des zylindrischen Innenraums zumindest genauso lang ist wie der Durchmesser eines am dichtesten in Richtung des Kartuschenkopfs angeordneten vorderen Dichtungsrings des zumindest einen Dichtungsrings, bevorzugt mindestens doppelt so lang ist wie der vordere Dichtungsring, oder wobei die zumindest eine Nut parallel zur Zylinderachse des zylindrischen Innenraums zumindest halb so lang ist wie der vordere Dichtungsring und entlang des Zylindermantels zumindest 4 mm breit ist, vorzugsweise zumindest 8 mm breit ist.

Hierdurch wird eine besonders einfache und kostengünstig zu realisierende Druckentlastungsvorrichtung realisiert, die automatisch durch Überfahren der zumindest einen Nut mit dem Dichtungsring oder dem vorderen Dichtungsring des Antriebskolbens aktiviert wird.

In einer weiteren Ausgestaltungsform der Vorrichtung kann vorgesehen sein, dass zur selbsttätigen beziehungsweise automatischen Druckentlastung an der Innenwand der Kartusche zu mindestens eine tangentiale Nut angeordnet ist, die eine axiale Erstreckung hat, die mindestens die Hälfte der Höhe des Dichtungsrings der Antriebskolbens hat. Das bedeutet, dass der Antriebskolben beim Auffahren auf den Mittelkolben an diesen an einer definierten axialen Position innerhalb der Kartusche aufläuft. Dabei fährt der Dichtungsring des Antriebskolbens auf die tangentiale Nut. Die vor dem Antriebskolben und hinter der Unterseite des Mittelkolbens stehende restliche Monomerflüssigkeit steht unter Druck.

Wenn der Dichtungsring beziehungsweise der vordere Dichtungsring des Antriebskolbens auf die Nut fährt, kann sich der Dichtungsring in die Nut ausdehnen und die Druckspannung des Dichtungsrings sinkt. Dadurch kann die unter Druck stehende Monomerflüssigkeit um den im Bereich der Nut befindliche Dichtungsring umfließen. Der Druck der Monomerflüssigkeit wird dadurch abgebaut. Vorteilhaft ist es, wenn in einem Abstand von mindestens 5 mm ein zweiter Dichtungsring am Antriebskolben angebracht ist. Dann wird die ausgetretene Monomerflüssigkeit in dem Raum zwischen der Mantelfläche des Antriebskolbens und der Innenwand der Kartusche gesammelt, der durch den oberen und den unteren Dichtungsring begrenzt wird. Die Mantelfläche des Antriebskolbens kann vorteilhaft eine oder mehrere Nuten enthalten, um die beim Überfahren des oberen Dichtungsringes ausgetretene Monomerflüssigkeit aufzunehmen.

Es kann auch vorgesehen sein, dass die Druckentlastungsvorrichtung durch einen komprimierbaren Antriebskolben und/oder durch einen komprimierbaren Mittelkolben realisiert ist und/oder die Druckentlastungsvorrichtung eine Expansion der Wandung der Kartusche im Bereich des zweiten Hohlraums ermöglicht und/oder eine auf die Zylinderachse des zylindrischen Innenraums bezogene axiale Verlängerung im Bereich des zweiten Hohlraums ermöglicht.

Hierdurch kann die Druckentlastungsvorrichtung im Inneren der Vorrichtung angeordnet werden und verbraucht zumindest außerhalb der Kartusche keinen zusätzlichen Platz. Dadurch ist die Druckentlastungvorrichtung für Störungen und Beschädigungen durch externe mechanische Einwirkungen unanfällig.

Der Erfindung liegt die überraschende Erkenntnis zugrunde, dass es durch eine Druckentlastung der Monomerflüssigkeit in dem zweiten Hohlraum nach dem Benetzen des Zementpulvers gelingt, im weiteren Verfahren auch bei einem Druck auf den Antriebskolben ein Nachschießen von Monomerflüssigkeit in den Knochenzement zu vermeiden und dadurch eine Verbesserung der Homogenität des hergestellten Knochenzements zu erreichen, auch wenn Antriebskolben mit großem Durchmesser von mehr als 25 mm verwendet werden. Die Erfindung basiert auch auf der überraschenden Erkenntnis, dass es insbesondere bei Antriebskolben mit großem Durchmesser zu derart großen hydrostatischen Kräften durch den auf der Monomerflüssigkeit lastenden Druck kommen kann, dass sich Monomerflüssigkeit in das bereits benetzte und angequollene Zementpulver pressen kann und dort zu Monomerflüssigkeitseinschlüssen im Knochenzement oder zu einem Knochenzement mit einer abweichenden Konsistenz führt.

Die Erfindung basiert auf der Beobachtung, dass bei denen gemäß der EP 3 320 869 B1 und EP 3 320 870 B1 gefertigten Zementiersystemen nach dem Auffahren des Antriebskolbens auf die zerborstenen Reste des Monomerflüssigkeitsbehälters und dem Mittelkolben durch radiale Deformation der Kartuschenwand eine Rückstellkraft auf die Monomerflüssigkeitsreste in dem Raum zwischen der Oberseite des Antriebskolben und der Unterseite des Mittelkolbens ausgeübt wird. Dadurch steht die restliche Monomerflüssigkeit in diesem Raum unter Druck. Es zeigte sich überraschend in praktischen Versuchen, dass nach erfolgter Vermischung des Zementpulvers mit der Monomerflüssigkeit eine Druckentlastung des Hohlraums zwischen der Oberseite des Antriebskolbens und der Unterseite des Mittelkolbens durch zumindest teilweises Entfernen von restlicher, eingeschlossener Monomerflüssigkeit das Einspritzen kleinster Monomerflüssigkeitsvolumina in den sich bereits bildenden oder in den bereits gebildeten Knochenzement verhindert.

Ein beispielhaftes und erfindungsgemäß bevorzugtes Verfahren zum Herstellen eines Knochenzements kann mit einer Vorrichtung zum Herstellen eines Knochenzements durchgeführt werden, die Vorrichtung aufweisend
a) eine hohlzylinderförmige Kartusche,
b) einen Kartuschenkopf mit einer Austragsöffnung,
c) einen für Gase und Flüssigkeiten durchlässigen und für Pulverpartikel undurchlässigen Verschluss, der in der Austragsöffnung axial beweglich angeordnet ist,
d) einen für Gase und Flüssigkeiten durchlässigen und für Pulverpartikel undurchlässigen Mittelkolben, der axial beweglich im Innenraum der Kartusche angeordnet ist,
e) einen ersten Hohlraum, der durch die Innenwand der Kartusche, den Verschluss und die Oberseite des Mittelkolbens begrenzt ist,
f) verdichtetes Knochenzementpulver im ersten Hohlraum,
g) einen unterhalb des Mittelkolbens angeordneten Antriebskolben, der für Gase und Flüssigkeiten undurchlässig ist und der axial im Innenraum der Kartusche axial beweglich ist,
h) einen zweiten Hohlraum, der durch die Unterseite des Mittelkolbens, die Innenwand der Kartusche und die Oberseite des Antriebskolbens begrenzt ist und
i) einem Monomerflüssigkeitsbehälter, der im zweiten Hohlraum angeordnet ist,
wobei das Verfahren durch die folgenden nacheinander ablaufenden Schritte gekennzeichnet ist
A) Bewegung des Antriebskolbens in Richtung des Kartuschenkopfs durch eine äußere Antriebsvorrichtung,
B) Zerstörung des Monomerflüssigkeitsbehälters durch die axiale Bewegung des Antriebskolbens in Richtung Kartuschenkopf,
C) Auspressen der überstehenden Luft durch den Mittelkolben, durch das Zementpulver und durch den Verschluss in die umgebende Atmosphäre,
D) Einpressen der Monomerflüssigkeit durch den Mittelkolben in das Zementpulver durch die weitere axiale Bewegung des Antriebskolbens in Richtung Kartuschenkopf,
E) Verdrängen der Luft zwischen den Zementpulverpartikeln,
F) vollständiges Benetzen der Zementpulverpartikel,
G) Druckentlastung des mit dem zerborstenen Monomerflüssigkeitsbehälters und mit Resten der Monomerflüssigkeit gefüllten zweiten Hohlraums, der durch die Unterseite des Mittelkolbens, der Oberseite des Antriebskolbens und der Innenwand der Kartusche begrenzt ist, durch zu mindestens teilweises Entfernen der in dem zweiten Hohlraum eingeschlossenen Monomerflüssigkeit,
H) Entfernen des Verschlusses aus dem Kartuschenkopf und
I) Auspressen des Knochenzements durch die axiale Bewegung des Antriebskolbens und des Mittelkolbens in Richtung des Kartuschenkopfs.

Erfindungsgemäß kann vorgesehen sein, dass die Druckentlastung durch manuelle Betätigung einer Druckentlastungsvorrichtung oder durch eine selbsttätige Druckentlastungsvorrichtung erfolgt.

Bei der manuellen Druckentlastung wird die Monomerflüssigkeit in ein Reservoir außerhalb der Kartusche abgeleitet. Bevorzugt wird dabei ein vollständiger Druckausgleich mit der umgebenden Atmosphäre hergestellt.

Bei der selbsttätigen oder automatischen Druckentlastung wird die Monomerflüssigkeit in ein Reservoir innerhalb oder unterhalb des Antriebskolbens und/oder einem Reservoir an der Seite des Antriebskolbens geleitet. Dabei wird ein teilweiser oder auch ein vollständiger Druckausgleich mit der umgebenden Atmosphäre erreicht.

Die beispielhafte erfindungsgemäße Vorrichtung zur Herstellung von Knochenzement, insbesondere zur Vermischung und zum Austragen von Polymethylmethacrylat-Knochenzement, kann aufweisen
a) eine hohlzylinderförmige Kartusche,
b) einen Kartuschenkopf mit einer Austragsöffnung,
c) einen für Gase und Flüssigkeiten durchlässigen und für Pulverpartikel des Zementpulvers undurchlässigen Verschluss, der in der Austragsöffnung axial beweglich angeordnet ist,
d) einen für Gase und Flüssigkeiten durchlässigen und für Pulverpartikel undurchlässigen Mittelkolben, der axial beweglich im Innenraum der Kartusche angeordnet ist,
e) einen ersten Hohlraum, der durch die Innenwand der Kartusche, den Verschluss und die Oberseite des Mittelkolbens begrenzt ist,
f) verdichtetes Knochenzementpulver im ersten Hohlraum,
g) einen unterhalb des Mittelkolbens angeordneten Antriebskolben, der für Gase und Flüssigkeiten undurchlässig ist und der im Innenraum der Kartusche axial beweglich ist,
h) einen zweiten Hohlraum, der durch die Unterseite des Mittelkolbens, die Innenwand der Kartusche und die Oberseite des Antriebskolbens begrenzt ist,
i) einen Monomerflüssigkeitsbehälter, der im zweiten Hohlraum angeordnet ist und
j) eine Druckentlastungsvorrichtung, mit der Monomerflüssigkeit aus dem zweiten Hohlraum zwischen Unterseite des Mittelkolbens und der Oberseite des Antriebskolbens ausgeleitet werden kann.

Im Folgenden werden weitere Ausführungsbeispiele der Erfindung anhand von siebzehn schematisch dargestellten Figuren erläutert, ohne jedoch dabei die Erfindung zu beschränken. Dabei zeigt:
Figur 1: eine schematische Querschnittansicht einer ersten beispielhaften erfindungsgemäßen Vorrichtung zum Herstellen eines Knochenzements;
Figur 2: eine schematische perspektivische Querschnittansicht der ersten beispielhaften erfindungsgemäßen Vorrichtung nach Figur 1;
Figur 3: eine schematische perspektivische Außenansicht der Vorrichtung nach den Figuren 1 und 2;
Figur 4: eine Ausschnittvergrößerung der Querschnittansicht der ersten beispielhaften erfindungsgemäßen Vorrichtung nach Figur 1;
Figur 5: eine schematische perspektivische Außenansicht einer zweiten beispielhaften erfindungsgemäßen Vorrichtung zum Herstellen eines Knochenzements;
Figur 6: eine schematische Querschnittansicht der zweiten beispielhaften erfindungsgemäßen Vorrichtung nach Figur 5;
Figur 7: eine Ausschnittvergrößerung der Querschnittansicht der zweiten beispielhaften erfindungsgemäßen Vorrichtung nach Figur 5;
Figur 8: eine schematische perspektivische Außenansicht einer dritten beispielhaften erfindungsgemäßen Vorrichtung zum Herstellen eines Knochenzements;
Figur 9: eine schematische Querschnittansicht der dritten beispielhaften erfindungsgemäßen Vorrichtung nach Figur 8;
Figur 10: eine Ausschnittvergrößerung der Querschnittansicht der dritten beispielhaften erfindungsgemäßen Vorrichtung nach Figur 8;
Figur 11: eine schematische perspektivische Außenansicht einer vierten beispielhaften erfindungsgemäßen Vorrichtung zum Herstellen eines Knochenzements;
Figur 12: eine schematische Querschnittansicht der vierten beispielhaften erfindungsgemäßen Vorrichtung nach Figur 11;
Figur 13: eine schematische perspektivische Außenansicht einer fünften beispielhaften erfindungsgemäßen Vorrichtung zum Herstellen eines Knochenzements;
Figur 14: eine schematische Querschnittansicht der fünften beispielhaften erfindungsgemäßen Vorrichtung nach Figur 13;
Figur 15: eine schematische perspektivische Querschnittansicht der fünften beispielhaften erfindungsgemäßen Vorrichtung nach Figur 13 und 14;
Figur 16: eine schematische perspektivische Außenansicht einer sechsten beispielhaften erfindungsgemäßen Vorrichtung zum Herstellen eines Knochenzements; und
Figur 17: eine schematische Querschnittansicht der sechsten beispielhaften erfindungsgemäßen Vorrichtung nach Figur 16.

Bei den Ausführungsbeispielen werden für gleichartige Bereiche, Teile und Bauteile unterschiedlicher Vorrichtungen zum Teil die gleichen Bezugszeichen verwendet, um die Vergleichbarkeit der Ausführungsbeispiele zu verbessern und die Lesbarkeit zu vereinfachen. Die unterschiedlichen Ausführungsbeispiele der unterschiedlichen Vorrichtungen sind dennoch als komplett voneinander getrennte Vorrichtungen zu verstehen, die nicht durch einen Umbau ineinander umgewandelt werden.

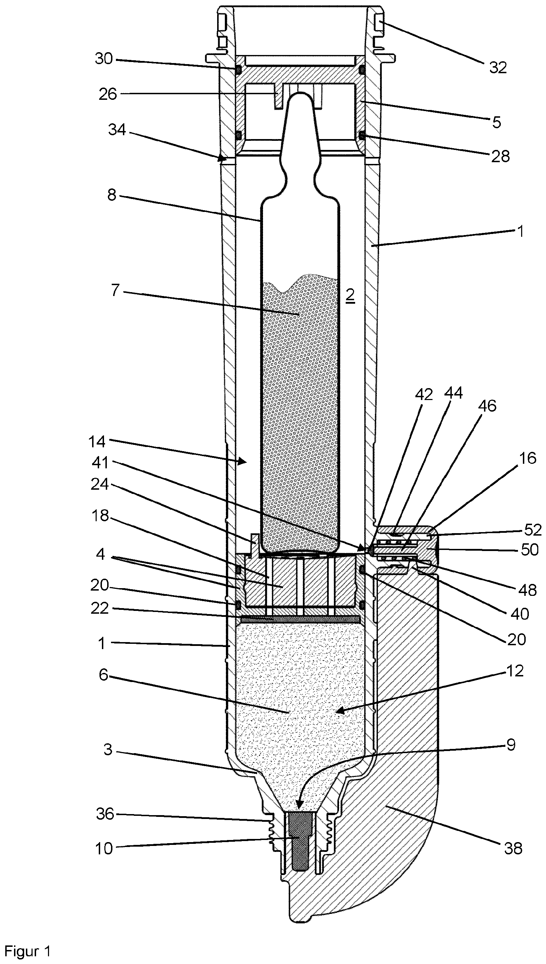

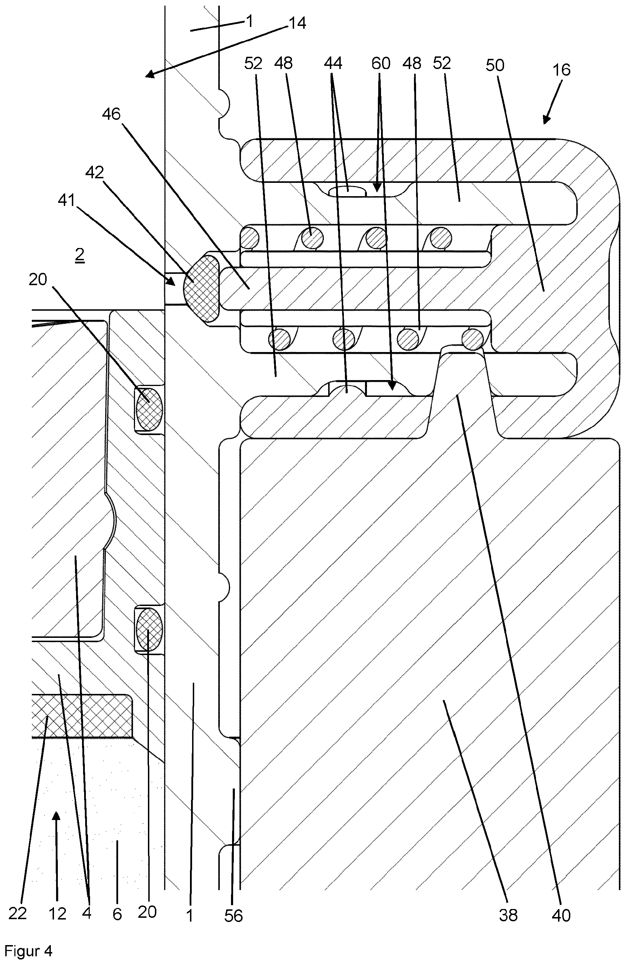

In den Figuren 1 bis 4 sind Abbildungen einer ersten erfindungsgemäßen Vorrichtung zum Herstellen eines Knochenzements gezeigt. Die Figuren 1 bis 3 zeigen verschiedene schematische Gesamtansichten der ersten beispielhaften erfindungsgemäßen Vorrichtung und Figur 4 eine Ausschnittvergrößerung der Querschnittansicht nach Figur 1.

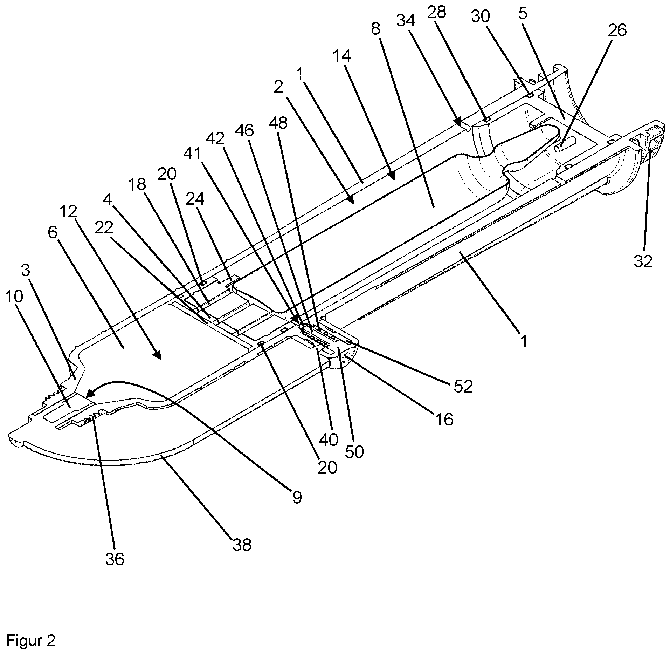

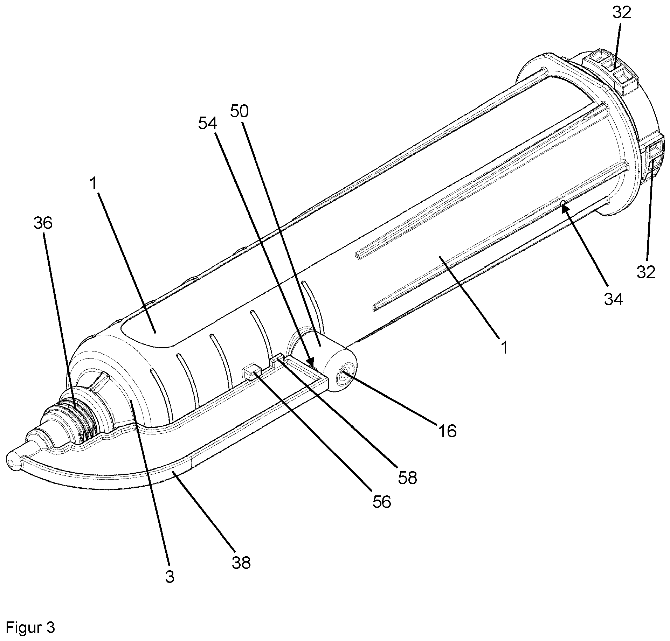

Gemäß dem ersten Ausführungsbeispiel der vorliegenden Erfindung kann die Vorrichtung eine röhrenförmige Kartusche 1 aus einem Kunststoff mit einem zylindrischen Innenraum 2 aufweisen, der an seiner Vorderseite (in Figur 1 unten und in den Figuren 2 und 3 links unten) durch einen Kartuschenkopf 3 geschlossen ist. Der Kartuschenkopf 3 besteht vorzugsweise ebenfalls aus einem Kunststoff. Im zylindrischen Innenraum 2 der Kartusche 1 kann ein in Richtung des Kartuschenkopfs 3 (in axialer Richtung) beweglich gelagerter Mittelkolben 4 und ein in Richtung des Kartuschenkopfs 3 (in axialer Richtung) beweglich gelagerter Antriebskolben 5 angeordnet sein.

In einem ersten Hohlraum 12 in der Kartusche 1, der an seiner Vorderseite durch den Kartuschenkopf 3 und an seiner Rückseite durch die Vorderseite des Mittelkolbens 4 begrenzt ist, ist ein Zementpulver 6 enthalten. Das Zementpulver 6 kann in dem ersten Hohlraum 12 eingepresst sein und diesen vollständig (das heißt bis auf die Zwischenräume zwischen den Pulverpartikeln des Zementpulvers 6) ausfüllen. Besonders bevorzugt ist das Zementpulver 6 in dem ersten Hohlraum 12 komprimiert und kann auch unter Druck stehen. In einem zweiten Hohlraum 14 in der Kartusche 1, der an seiner Vorderseite durch die Rückseite des Mittelkolbens 4 und an seiner Rückseite durch die Vorderseite des Antriebskolbens 5 begrenzt ist, kann eine Monomerflüssigkeit 7 in einem Monomerflüssigkeitsbehälter 8 angeordnet sein. Der Monomerflüssigkeitsbehälter 8 kann eine Glasampulle oder eine Kunststoffampulle sein, die in dem zweiten Hohlraum 14 durch Vortreiben des Antriebskolbens 6 aufbrechbar ist. Der Mittelkolben 4 stützt sich dabei auf dem im ersten Hohlraum 12 enthaltenen Zementpulver 6 ab. Das Zementpulver 6 wird erst fließfähig, wenn es durchgehend oder vollständig mit der Monomerflüssigkeit 7 benetzt ist.

Im Kartuschenkopf 3 ist eine Austragsöffnung 9 angeordnet, durch die ein aus dem Zementpulver 6 und der Monomerflüssigkeit 7 hergestellter Knochenzement aus dem ersten Hohlraum 12 ausgepresst werden kann. Die Austragsöffnung 9 kann aber zunächst mit einem Verschluss 10 verschlossen sein, der für Gase durchlässig ist aber für das Zementpulver 6 undurchlässig ist. Die Austragsöffnung 9 kann zentral in dem Kartuschenkopf 3 angeordnet sein. Der Kartuschenkopf 3 kann sich in Richtung der Austragsöffnung 9 verjüngen, um den Austrag des Knochenzements aus dem ersten Hohlraum 12 heraus zu erleichtern.

Der Monomerflüssigkeitsbehälter 8 kann durch Vortreiben des Antriebskolbens 5 aufgebrochen werden und dadurch die Monomerflüssigkeit 7 in dem zweiten Hohlraum 14 freigesetzt werden. Anschließend kann durch weiteres Vortreiben des Antriebskolbens 5 die Monomerflüssigkeit 7 in das Zementpulver 6 im ersten Hohlraum 12 gepresst werden. Durch eine Spannung der Wandungen der Kartusche 1 und durch elastische Verformungen der den zweiten Hohlraum 14 begrenzenden Teile der Vorrichtung steht die Monomerflüssigkeit 7 im zweiten Hohlraum 12 anschließend unter Druck.

Die Vorrichtung hat eine Druckentlastungsvorrichtung 16, mit der dieser Druck in dem zweiten Hohlraum 14 durch Ableiten von Teilen der Monomerflüssigkeit 7 aus dem zweiten Hohlraum 12 reduziert werden kann. Die Druckentlastungsvorrichtung 16 ist an einer seitlichen Oberfläche der Kartusche 1 angeordnet. Theoretisch können auch mehrere gleichartige oder auch unterschiedliche Druckentlastungsvorrichtungen vorgesehen sein, um den Druck der Monomerflüssigkeit 7 in dem zweiten Hohlraum 12 abzubauen. Die Druckentlastungsvorrichtung 16 ist als Querschnitt im Detail in der Ausschnittvergrößerung nach Figur 4 gezeigt.

In dem Mittelkolben 4 können mehrere Leitungen 18 angeordnet sein, durch die die Monomerflüssigkeit 7 aus dem zweiten Hohlraum 14 in den ersten Hohlraum 12 gepresst werden kann. Der Mittelkolben 4 kann ansonsten gegen die Innenwand der Kartusche 1 mit Hilfe umlaufender ringförmiger Dichtungen 20 abgedichtet sein. Die Dichtungen 20 können als O-Ringe ausgeführt sein und aus einem elastischen Kunststoff wie Gummi bestehen. Ein Porenfilter 22 kann die Leitungen 18 an der Vorderseite des Mittelkolbens 4 abdecken. Der Porenfilter 22 ist für die Monomerflüssigkeit 7 und Gase durchlässig aber für das Zementpulver 6 undurchlässig. Dadurch wird verhindert, dass das Zementpulver 6 in die Leitungen 18 eindringen kann und diese verschließt, wenn es darin mit der Monomerflüssigkeit 7 in Kontakt kommt. An der Rückseite des Mittelkolbens 4 können mehrere Stifte 24 zur Zentrierung und Positionierung des Monomerflüssigkeitsbehälters 8 angeordnet sein. Ebenso können mehrere Stifte 26 zur Zentrierung und Lagerung des Monomerflüssigkeitsbehälters 8 an der Vorderseite des Antriebskolbens 5 angeordnet sein.

Der Antriebskolben 5 kann eine vordere Dichtung 28 und eine hintere Dichtung 30 aufweisen, mit der der Antriebskolben 5 gegen die Innenwand der Kartusche 1 abgedichtet ist. Die vordere Dichtung 28 und die hintere Dichtung 30 können als O-Ringe ausgeführt sein und aus einem elastischen Kunststoff wie Gummi bestehen. Der Antriebskolben 5 ist auf diese Weise für die Monomerflüssigkeit 7 und vorzugsweise auch für Gase undurchlässig und liegt auch undurchlässig für die Monomerflüssigkeit 7 und Gase an der Innenwand der Kartusche 1 an.

An der Rückseite der Kartusche 1 kann außen ein Befestigungsmittel 32 zur Befestigung einer Auspressvorrichtung (nicht gezeigt) angeordnet sein. Der Antriebskolben 5 kann mit einem Stößel einer angeschlossenen Auspressvorrichtung vorgetrieben beziehungsweise angetrieben werden, um ein erfindungsgemäßes Verfahren umzusetzen.

In der Kartuschenwand der Kartusche 1 können Begasungsöffnungen 34 angeordnet sein. Diese liegen unmittelbar vor dem Antriebskolben 5 in seiner Ausgangsstellung, die in Figur 1 gezeigt ist. Durch die Begasungsöffnungen 34 ist der Innenraum 2 der Kartusche 1 für ein sterilisierendes Gas wie Ethylenoxid zugänglich. Dadurch kann die Vorrichtung sterilisiert werden und steril verpackt und aufbewahrt werden, was für eine medizinische Anwendung des damit hergestellten Knochenzements wichtig ist. Die Begasungsöffnungen 34 werden vorzugsweise von dem Antriebskolben 5 überfahren, bevor der Monomerflüssigkeitsbehälter 8 geöffnet ist, so dass die freigesetzte Monomerflüssigkeit 7 nicht durch die Begasungsöffnungen 34 austreten kann.

An der Vorderseite der Kartusche 1 kann am Kartuschenkopf 3 ein Stutzen mit einem Außengewinde 36 angeordnet sein, der die Austragsöffnung 9 begrenzt und verlängert. An das Außengewinde 36 kann bei Bedarf ein Austragsrohr (nicht gezeigt) mit einem passenden Innengewinde angeschlossen werden.

Der Verschluss 10 kann mit einem Verbindungselement 38 aus Kunststoff verbunden sein. Das Verbindungselement 38 kann einen Bügel bilden, der sich von dem Verschluss 10 bis zu einem Zapfen 40 erstreckt. Der Zapfen 40 dient als Arretierung der Druckentlastungsvorrichtung 16. Wenn der Verschluss 10 aus der Austragsöffnung 9 herausgezogen wird oder herausgedrückt wird, wird über das Verbindungselement 38 automatisch auch der Zapfen 40 aus der Druckentlastungsvorrichtung 16 gezogen. Dadurch kann die Druckentlastungsvorrichtung 16 automatisch durch Entfernen des Verschlusses 10 aktiviert werden.

In der Wandung der Kartusche 1 kann eine durchgehende Verbindung 41 vorgesehen sein, durch die die Druckentlastungsvorrichtung 16 mit dem zweiten Hohlraum 14 für die Monomerflüssigkeit 7 durchlässig verbunden oder verbindbar ist. Theoretisch können auch mehrere durchgehende Verbindungen 41 vorgesehen sein, die an die gleiche Druckentlastungsvorrichtung 16 oder auch an mehrere gleichartige oder unterschiedliche Druckentlastungsvorrichtungen angeschlossen sind. Zum Verschließen der durchgehenden Verbindung 41 kann ein Dichtungskörper 42 auf die Verbindung der Druckentlastungsvorrichtung 16 zur durchgehenden Verbindung 41 gedrückt werden. Der Dichtungskörper 42 kann beispielsweise durch einen Gummipfropfen realisiert werden. Der Dichtungskörper 42 kann mit einer Federführungsstange 46 mit Hilfe einer Kappe 50 in den Sitz gepresst werden. Eine Feder 48 kann die Kappe 50 von der Kartusche 1 und damit den Dichtungskörper 42 von der durchgehenden Verbindung 41 wegdrücken. Die Federführungsstange 46 kann innen an einer Kappe 50 der Druckentlastungsvorrichtung 16 angeordnet sein. Die Feder 48 kann unter Druck stehen und sich auf einer Seite außen an der Kartusche 1 und auf der anderen Seite innen an der Kappe 50 abstützen. Die Feder 48 kann um die Federführungsstange 46 herum gewunden sein. Die durchgehende Verbindung 41 kann direkt neben dem Mittelkolben 4 in den zweiten Hohlraum 14 münden. Dadurch kann sichergestellt werden, dass der Antriebskolben 5 die Einmündung in die durchgehende Verbindung 41 nicht verschließt, wenn er in Richtung des Mittelkolbens 4 gedrückt ist.

Die Druckentlastungsvorrichtung 16 kann ferner einen hohlen Stutzen 52 aufweisen, der vorzugsweise einteilig mit der Kartusche 1 ausgeführt ist. Der Zapfen 40 kann sich durch einen Durchgang 54 in der Kappe 50 und den hohlen Stutzen 52 erstrecken. Dadurch kann die Kappe 50 mit dem Zapfen 40 lösbar an dem hohlen Stutzen 52 fixiert werden und die Feder 48 im komprimierten Zustand gehalten werden. Wenn der Zapfen 40 aus dem Durchgang 54 herausgezogen wird, drückt die Feder 48 die Kappe 50 von der Kartusche 1 weg und der Dichtungskörper 42 löst sich von der durchgehenden Verbindung 41. Dadurch kann in dem zweiten Hohlraum 14 unter Druck stehende Monomerflüssigkeit 7 in die Druckentlastungsvorrichtung 16 strömen und dadurch der hydrostatische Druck der Monomerflüssigkeit 7 in dem zweiten Hohlraum 14 reduziert werden.

Damit die Monomerflüssigkeit 7 nicht in die Umgebung der Vorrichtung gelangt, kann die Kappe 50 dicht aber gleichzeitig beweglich mit dem Stutzen 52 verbunden sein. Hierzu können an der Kappe 50 Vorsprünge 44 und an dem Stutzen 52 eine Nut 60 vorgesehen sein, wobei die Vorsprünge 44 in die Nut 60 greifen und die Nut 60 so lang ist, dass die Vorsprünge 44 sich in der Nut 60 bewegen können und dass die Kappe 50 sich so weit von der Kartusche 1 abheben kann, dass sich der Dichtungskörper 42 von der durchgehenden Verbindung 41 lösen kann, aber nicht so weit, dass sich die Kappe 50 von dem Stutzen 52 löst oder die Kappe 50 gegen den Stutzen 52 undicht wird. Alternativ könnten auch die Nut 60 in der Kappe 50 und die Vorsprünge 44 an dem Stutzen 52 angeordnet werden. Hierdurch bildet die Druckentlastungsvorrichtung 16 in ihrem Inneren ein Reservoir zur Aufnahme von Monomerflüssigkeit 7, wobei ein Zellstoff, eine Zellulose oder ein saugfähiges Material (nicht gezeigt) in dem Reservoir angeordnet sein kann, mit dem die Monomerflüssigkeit 7 aufgesaugt und gebunden werden kann.

An der Außenseite der Kartusche 1 kann eine Führung 56 zur Führung des Verbindungselements 38 angeordnet sein. Ein Stopper 58 kann an dem Verbindungselement 38 angeordnet sein. Wenn der Knochenzement in dem ersten Hohlraum 12 aufquillt und sich ausdehnt oder mit dem Mittelkolben 4 in die Austragsöffnung 9 gedrückt wird, wird der Verschluss 10 nach vorne (in Figur 1 nach unten) gedrückt und dabei das Verbindungselement 38 nach vorne gedrückt, bis der Stopper 58 an der Führung 56 anschlägt und eine weitere Bewegung blockiert. Der Zapfen 40 wird dadurch aus dem Durchgang 54 gezogen und die Druckentlastungsvorrichtung 16 aktiviert, ohne dass der Verschluss 10 bereits vollständig aus der Austragsöffnung 9 entfernt werden muss. Der Anwender erkennt daran, dass der Knochenzement gemischt und die Vorrichtung bereit ist, den Knochenzement abzugeben. Dann kann der Verschluss 10 entfernt werden und gegebenenfalls ein Austragsrohr an das Außengewinde 36 angeschlossen werden.

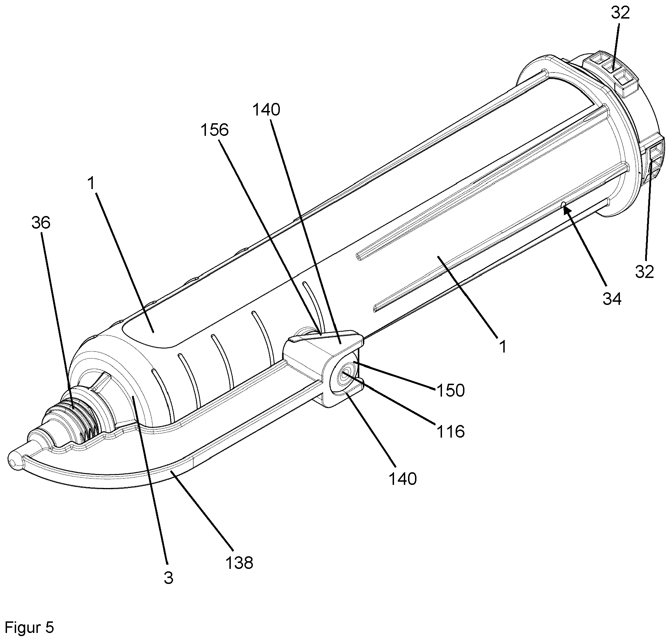

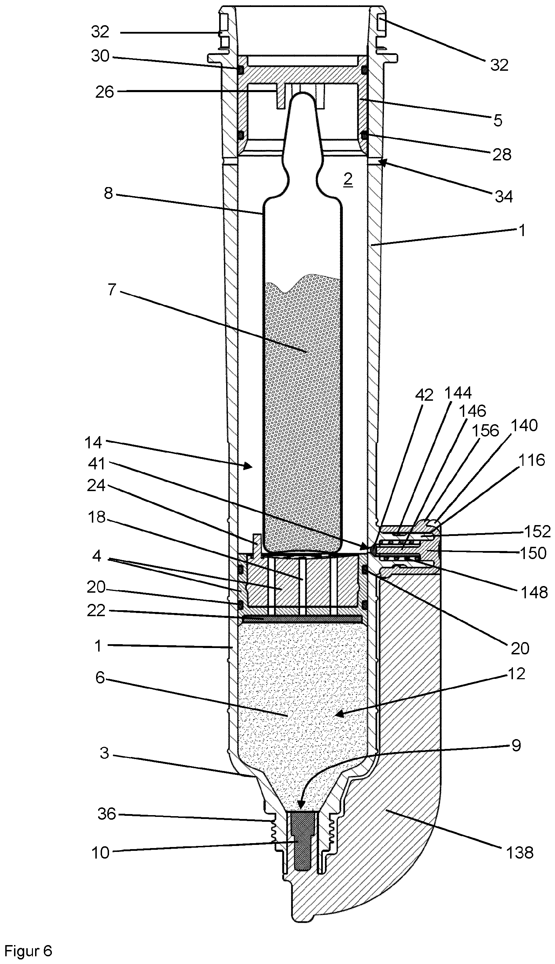

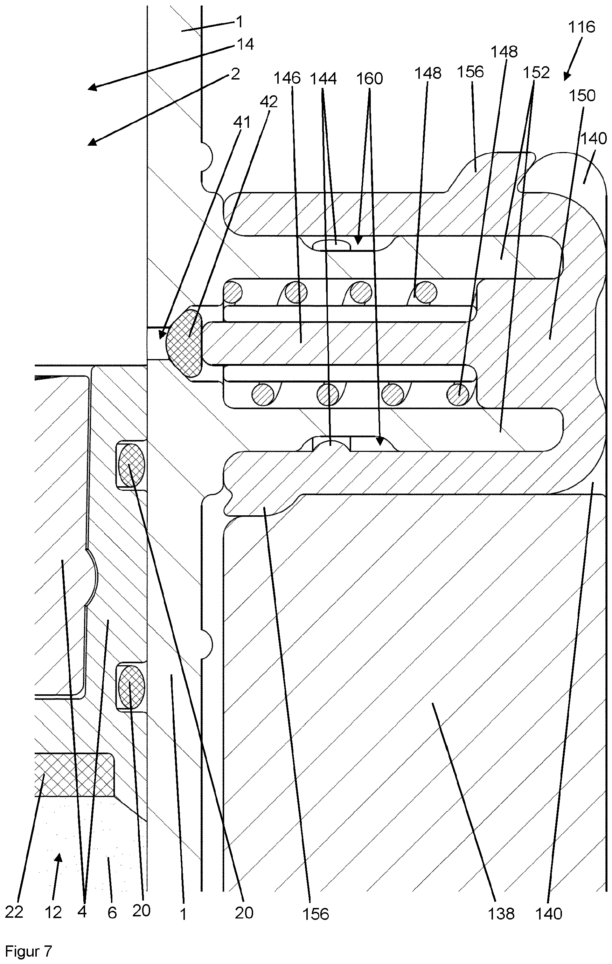

In den Figuren 5 bis 7 sind Abbildungen einer zweiten erfindungsgemäßen Vorrichtung zum Herstellen eines Knochenzements gezeigt. Die Figuren 5 und 6 zeigen verschiedene schematische Gesamtansichten der zweiten beispielhaften erfindungsgemäßen Vorrichtung und Figur 7 eine Ausschnittvergrößerung der Querschnittansicht nach Figur 6.

Auch die Vorrichtung gemäß dem zweiten Ausführungsbeispiel der vorliegenden Erfindung kann eine röhrenförmige Kartusche 1 aus einem Kunststoff mit einem zylindrischen Innenraum 2 aufweisen, der an seiner Vorderseite (in Figur 5 links unten und in Figur 6 unten) durch einen Kartuschenkopf 3 geschlossen ist. Der Kartuschenkopf 3 besteht vorzugsweise ebenfalls aus einem Kunststoff. Im zylindrischen Innenraum 2 der Kartusche 1 kann ein in Richtung des Kartuschenkopfs 3 (in axialer Richtung) beweglich gelagerter Mittelkolben 4 und ein in Richtung des Kartuschenkopfs 3 (in axialer Richtung) beweglich gelagerter Antriebskolben 5 angeordnet sein.

In einem ersten Hohlraum 12 in der Kartusche 1, der an seiner Vorderseite durch den Kartuschenkopf 3 und an seiner Rückseite durch die Vorderseite des Mittelkolbens 4 begrenzt ist, ist ein Zementpulver 6 enthalten. Das Zementpulver 6 kann in dem ersten Hohlraum 12 eingepresst sein und diesen vollständig (das heißt bis auf die Zwischenräume zwischen den Pulverpartikeln des Zementpulvers 6) ausfüllen. Besonders bevorzugt ist das Zementpulver 6 in dem ersten Hohlraum 12 komprimiert und kann auch unter Druck stehen. In einem zweiten Hohlraum 14 in der Kartusche 1, der an seiner Vorderseite durch die Rückseite des Mittelkolbens 4 und an seiner Rückseite durch die Vorderseite des Antriebskolbens 5 begrenzt ist, kann eine Monomerflüssigkeit 7 in einem Monomerflüssigkeitsbehälter 8 angeordnet sein. Der Monomerflüssigkeitsbehälter 8 kann eine Glasampulle oder eine Kunststoffampulle sein, die in dem zweiten Hohlraum 14 durch Vortreiben des Antriebskolbens 6 aufbrechbar ist. Der Mittelkolben 4 stützt sich dabei auf dem im ersten Hohlraum 12 enthaltenen Zementpulver 6 ab. Das Zementpulver 6 wird erst fließfähig, wenn es durchgehend oder vollständig mit der Monomerflüssigkeit 7 benetzt ist.

Im Kartuschenkopf 3 ist eine Austragsöffnung 9 angeordnet, durch die ein aus dem Zementpulver 6 und der Monomerflüssigkeit 7 hergestellter Knochenzement aus dem ersten Hohlraum 12 ausgepresst werden kann. Die Austragsöffnung 9 kann aber zunächst mit einem Verschluss 10 verschlossen sein, der für Gase durchlässig ist aber für das Zementpulver 6 undurchlässig ist. Die Austragsöffnung 9 kann zentral in dem Kartuschenkopf 3 angeordnet sein. Der Kartuschenkopf 3 kann sich in Richtung der Austragsöffnung 9 verjüngen, um den Austrag des Knochenzements aus dem ersten Hohlraum 12 heraus zu erleichtern.

Der Monomerflüssigkeitsbehälter 8 kann durch Vortreiben des Antriebskolbens 5 aufgebrochen werden und dadurch die Monomerflüssigkeit 7 in dem zweiten Hohlraum 14 freigesetzt werden. Anschließend kann durch weiteres Vortreiben des Antriebskolbens 5 die Monomerflüssigkeit 7 in das Zementpulver 6 im ersten Hohlraum 12 gepresst werden. Durch eine Spannung der Wandungen der Kartusche 1 und durch elastische Verformungen der den zweiten Hohlraum 14 begrenzenden Teile der Vorrichtung steht die Monomerflüssigkeit 7 im zweiten Hohlraum 12 anschließend unter Druck. Insoweit gleicht der Aufbau der Vorrichtung nach dem zweiten Ausführungsbeispiel dem des ersten Ausführungsbeispiels.

Die Vorrichtung hat eine Druckentlastungsvorrichtung 116, mit der dieser Druck in dem zweiten Hohlraum 14 durch Ableiten von Teilen der Monomerflüssigkeit 7 aus dem zweiten Hohlraum 12 reduziert werden kann, die sich aber von der Druckentlastungsvorrichtung 16 des ersten Ausführungsbeispiels unterscheidet. Die Druckentlastungsvorrichtung 116 ist an einer seitlichen Oberfläche der Kartusche 1 angeordnet. Theoretisch können auch mehrere gleichartige oder auch unterschiedliche Druckentlastungsvorrichtungen vorgesehen sein, um den Druck der Monomerflüssigkeit 7 in dem zweiten Hohlraum 12 abzubauen. Die Druckentlastungsvorrichtung 116 ist als Querschnitt im Detail in der Ausschnittvergrößerung nach Figur 7 gezeigt.

In dem Mittelkolben 4 können mehrere Leitungen 18 angeordnet sein, durch die die Monomerflüssigkeit 7 aus dem zweiten Hohlraum 14 in den ersten Hohlraum 12 gepresst werden kann. Der Mittelkolben 4 kann ansonsten gegen die Innenwand der Kartusche 1 mit Hilfe umlaufender ringförmiger Dichtungen 20 abgedichtet sein. Die Dichtungen 20 können als O-Ringe ausgeführt sein und aus einem elastischen Kunststoff wie Gummi bestehen. Ein Porenfilter 22 kann die Leitungen 18 an der Vorderseite des Mittelkolbens 4 abdecken. Der Porenfilter 22 ist für die Monomerflüssigkeit 7 und Gase durchlässig aber für das Zementpulver 6 undurchlässig. Dadurch wird verhindert, dass das Zementpulver 6 in die Leitungen 18 eindringen kann und diese verschließt, wenn es darin mit der Monomerflüssigkeit 7 in Kontakt kommt. An der Rückseite des Mittelkolbens 4 können mehrere Stifte 24 zur Zentrierung und Positionierung des Monomerflüssigkeitsbehälters 8 angeordnet sein. Ebenso können mehrere Stifte 26 zur Zentrierung und Lagerung des Monomerflüssigkeitsbehälters 8 an der Vorderseite des Antriebskolbens 5 angeordnet sein.

Der Antriebskolben 5 kann eine vordere Dichtung 28 und eine hintere Dichtung 30 aufweisen, mit der der Antriebskolben 5 gegen die Innenwand der Kartusche 1 abgedichtet ist. Die vordere Dichtung 28 und die hintere Dichtung 30 können als O-Ringe ausgeführt sein und aus einem elastischen Kunststoff wie Gummi bestehen. Der Antriebskolben 5 ist auf diese Weise für die Monomerflüssigkeit 7 und vorzugsweise auch für Gase undurchlässig und liegt auch undurchlässig für die Monomerflüssigkeit 7 und Gase an der Innenwand der Kartusche 1 an.

An der Rückseite der Kartusche 1 kann außen ein Befestigungsmittel 32 zur Befestigung einer Auspressvorrichtung (nicht gezeigt) angeordnet sein. Der Antriebskolben 5 kann mit einem Stößel einer angeschlossenen Auspressvorrichtung vorgetrieben beziehungsweise angetrieben werden, um ein erfindungsgemäßes Verfahren umzusetzen.

In der Kartuschenwand der Kartusche 1 können Begasungsöffnungen 34 angeordnet sein. Diese liegen unmittelbar vor dem Antriebskolben 5 in seiner Ausgangsstellung, die in Figur 1 gezeigt ist. Durch die Begasungsöffnungen 34 ist der Innenraum 2 der Kartusche 1 für ein sterilisierendes Gas wie Ethylenoxid zugänglich. Dadurch kann die Vorrichtung sterilisiert werden und steril verpackt und aufbewahrt werden, was für eine medizinische Anwendung des damit hergestellten Knochenzements wichtig ist. Die Begasungsöffnungen 34 werden vorzugsweise von dem Antriebskolben 5 überfahren, bevor der Monomerflüssigkeitsbehälter 8 geöffnet ist, so dass die freigesetzte Monomerflüssigkeit 7 nicht durch die Begasungsöffnungen 34 austreten kann.

An der Vorderseite der Kartusche 1 kann am Kartuschenkopf 3 ein Stutzen mit einem Außengewinde 36 angeordnet sein, der die Austragsöffnung 9 begrenzt und verlängert. An das Außengewinde 36 kann bei Bedarf ein Austragsrohr (nicht gezeigt) mit einem passenden Innengewinde angeschlossen werden.

Der Verschluss 10 kann mit einem Verbindungselement 138 aus Kunststoff verbunden sein. Das Verbindungselement 138 kann einen Bügel bilden, der sich von dem Verschluss 10 bis zu einem gabelförmigen Stützkörper 140 erstreckt. Der gabelförmige Stützkörper 140 dient als Arretierung der Druckentlastungsvorrichtung 116. Wenn der Verschluss 10 aus der Austragsöffnung 9 herausgezogen wird oder herausgedrückt wird, wird über das Verbindungselement 138 automatisch auch der gabelförmige Stützkörper 140 von der Druckentlastungsvorrichtung 116 abgezogen. Dadurch kann die Druckentlastungsvorrichtung 116 automatisch durch Entfernen des Verschlusses 10 aktiviert werden.

In der Wandung der Kartusche 1 kann eine durchgehende Verbindung 41 vorgesehen sein, durch die die Druckentlastungsvorrichtung 116 mit dem zweiten Hohlraum 14 für die Monomerflüssigkeit 7 durchlässig verbunden oder verbindbar ist. Theoretisch können auch mehrere durchgehende Verbindungen 41 vorgesehen sein, die an die gleiche Druckentlastungsvorrichtung 116 oder auch an mehrere gleichartige oder unterschiedliche Druckentlastungsvorrichtungen angeschlossen sind. Zum Verschließen der durchgehenden Verbindung 41 kann ein Dichtungskörper 42 auf die Verbindung der Druckentlastungsvorrichtung 116 zur durchgehenden Verbindung 41 gedrückt werden. Der Dichtungskörper 42 kann beispielsweise durch einen Gummipfropfen realisiert werden. Der Dichtungskörper 42 kann mit einer Federführungsstange 146 mit Hilfe einer Kappe 150 in den Sitz gepresst werden. Eine Feder 148 kann die Kappe 150 von der Kartusche 1 und damit den Dichtungskörper 42 von der durchgehenden Verbindung 41 wegdrücken. Die Federführungsstange 146 kann innen an einer Kappe 150 der Druckentlastungsvorrichtung 116 angeordnet sein. Die Feder 148 kann unter Druck stehen und sich auf einer Seite außen an der Kartusche 1 und auf der anderen Seite innen an der Kappe 150 abstützen. Die Feder 148 kann um die Federführungsstange 146 herum gewunden sein. Die durchgehende Verbindung 41 kann direkt neben dem Mittelkolben 4 in den zweiten Hohlraum 14 münden. Dadurch kann sichergestellt werden, dass der Antriebskolben 5 die Einmündung in die durchgehende Verbindung 41 nicht verschließt, wenn er in Richtung des Mittelkolbens 4 gedrückt ist.

Die Druckentlastungsvorrichtung 116 kann ferner einen hohlen Stutzen 152 aufweisen, der vorzugsweise einteilig mit der Kartusche 1 ausgeführt ist. Der gabelförmige Stützkörper 140 kann außen auf einer vorspringenden Schiene 156 der Kappe 150 aufliegen und die Kappe 150 dadurch gegen die Kraft der Feder 148 in Richtung der Kartusche 1 gedrückt halten. Dadurch bleibt die Feder 148 komprimiert und der Dichtungskörper 42 in den Dichtungssitz gepresst und dadurch die durchgehende Verbindung 41 in die Druckentlastungsvorrichtung 116 verschlossen. Dadurch kann die Kappe 150 mit dem gabelförmigen Stützkörper 140 auf den hohlen Stutzen 152 gedrückt werden. Wenn der gabelförmige Stützkörper 140 entfernt wird, drückt die Feder 148 die Kappe 150 von der Kartusche 1 weg und der Dichtungskörper 42 löst sich von der durchgehenden Verbindung 41. Dadurch kann in dem zweiten Hohlraum 14 unter Druck stehende Monomerflüssigkeit 7 in die Druckentlastungsvorrichtung 116 strömen und dadurch der hydrostatische Druck der Monomerflüssigkeit 7 in dem zweiten Hohlraum 14 reduziert werden.

Damit die Monomerflüssigkeit 7 nicht in die Umgebung der Vorrichtung gelangt, kann die Kappe 150 dicht aber gleichzeitig beweglich mit dem Stutzen 152 verbunden sein. Hierzu können an der Kappe 150 Vorsprünge 144 und an dem Stutzen 152 eine Nut 160 vorgesehen sein, wobei die Vorsprünge 144 in die Nut 160 greifen und die Nut 160 so lang ist, dass die Vorsprünge 144 sich in der Nut 160 bewegen können und dass die Kappe 150 sich so weit von der Kartusche 1 abheben kann, dass sich der Dichtungskörper 42 von der durchgehenden Verbindung 41 lösen kann, aber nicht so weit, dass sich die Kappe 150 von dem Stutzen 152 löst oder die Kappe 150 gegen den Stutzen 152 undicht wird. Alternativ könnten auch die Nut 160 in der Kappe 150 und die Vorsprünge 144 an dem Stutzen 152 angeordnet werden. Hierdurch bildet die Druckentlastungsvorrichtung 116 in ihrem Inneren ein Reservoir zur Aufnahme von Monomerflüssigkeit 7, wobei ein Zellstoff, eine Zellulose oder ein saugfähiges Material (nicht gezeigt) in dem Reservoir angeordnet sein kann, mit dem die Monomerflüssigkeit 7 aufgesaugt und gebunden werden kann.

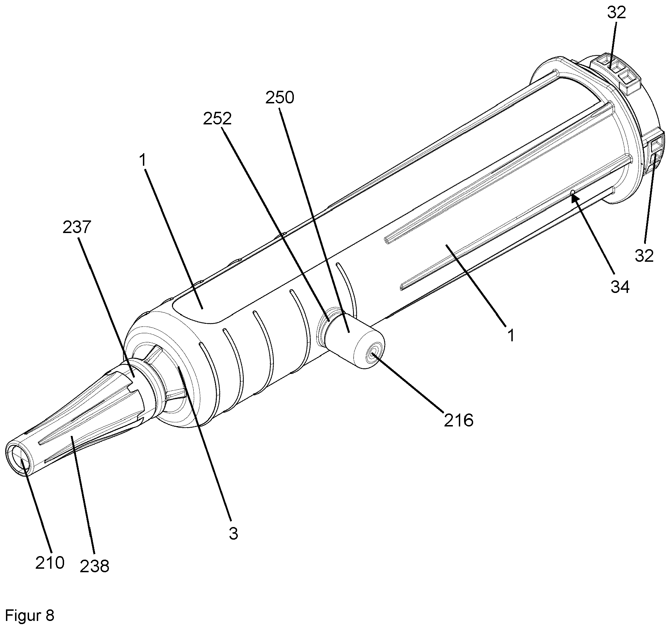

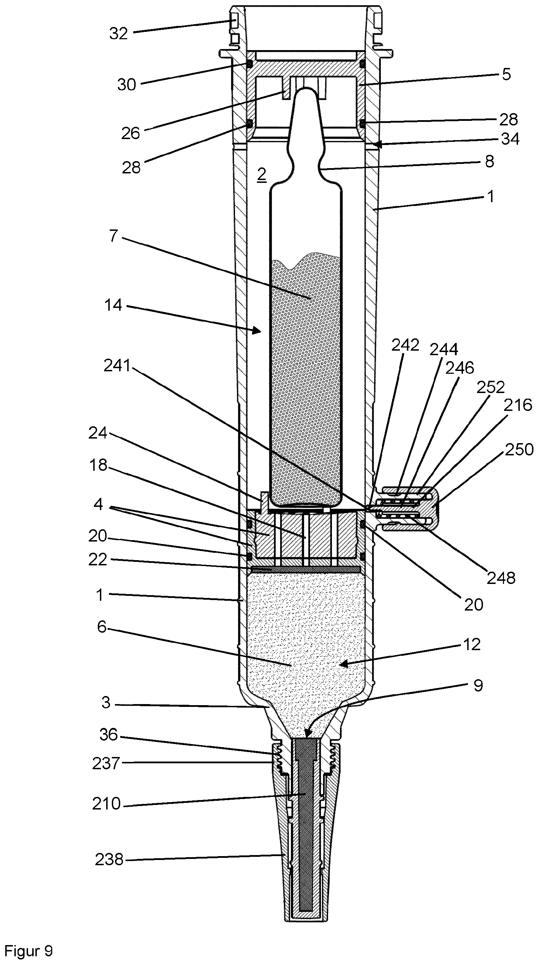

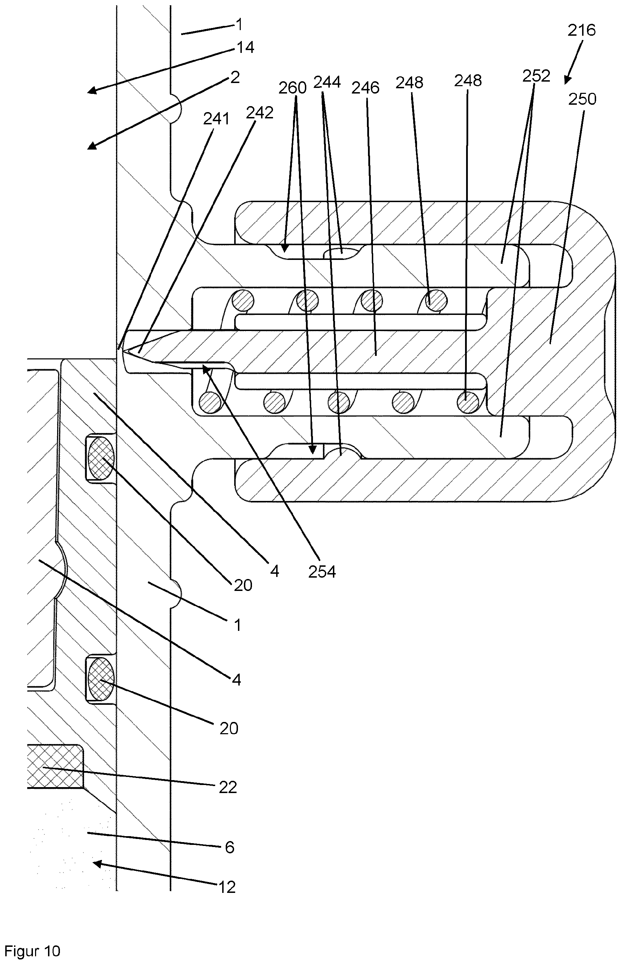

In den Figuren 8 bis 10 sind Abbildungen einer dritten erfindungsgemäßen Vorrichtung zum Herstellen eines Knochenzements gezeigt. Die Figuren 8 und 9 zeigen verschiedene schematische Gesamtansichten der dritten beispielhaften erfindungsgemäßen Vorrichtung und Figur 10 eine Ausschnittvergrößerung der Querschnittansicht nach Figur 9.

Der Aufbau der dritten erfindungsgemäßen Vorrichtung entspricht in weiten Teilen dem der ersten und der zweiten erfindungsgemäßen Vorrichtung. Die dritte erfindungsgemäße Vorrichtung kann dementsprechend ebenfalls eine Kartusche 1 mit einem zylindrischen Innenraum 2 aufweisen, die an ihrer Vorderseite (in Figur 8 links unten und in Figur 9 unten) mit einem Kartuschenkopf 3 enthaltend eine Austragsöffnung 9 geschlossen ist. Die dritte Vorrichtung weist dementsprechend ebenfalls einen Mittelkolben 4 und einen Antriebskolben 5 auf, die axial in dem zylindrischen Innenraum 2 der Kartusche 3 beweglich angeordnet sind. Auch die Anordnung von Zementpulver 6 und Monomerflüssigkeit 7 in einem Monomerflüssigkeitsbehälter 8 in den von dem Mittelkolben 4 getrennten ersten Hohlraum 12 und zweiten Hohlraum 14 des zylindrischen Innenraums 2 der Kartusche 1 ist analog dem ersten und dem zweiten Ausführungsbeispiel. Der Aufbau des Mittelkolbens 4 mit den Leitungen 18, den beiden Dichtungen 20, dem Porenfilter 22 und der Stifte 24 entspricht genauso dem des ersten und zweiten Ausführungsbeispiels wie der Aufbau des Antriebskolbens 5 mit den Stiften 26, der vorderen Dichtung 28 und der hinteren Dichtung 30. Ebenso können ein Befestigungsmittel 32, Begasungsöffnungen 34 und/oder ein Stutzen mit einem Außengewinde 36 an der Kartusche 1 entsprechend dem ersten und zweiten Ausführungsbeispiel angeordnet sein.

Insofern gleicht das dritte Ausführungsbeispiel dem ersten und dem zweiten Ausführungsbeispiel und für Details zum Aufbau und den Funktionen der entsprechenden Teile des dritten Ausführungsbeispiels wird zur Vermeidung von Wiederholungen auf die Beschreibung zum ersten und zweiten Ausführungsbeispiel verwiesen.

Die Austragsöffnung 9 kann zunächst mit einem Verschluss 210 verschlossen sein, der für Gase durchlässig ist aber für das Zementpulver 6 undurchlässig ist. Eine Kappe 238 kann über eine Schraubverbindung 237 mit dem Außengewinde 36 an der Vorderseite der Kartusche 1 verbunden sein. Der Verschluss 210 ist aus der Austragsöffnung 9 und aus der Kappe 238 herausdrückbar, so dass der Verschluss 210 nach vorne aus der Kappe 238 heraussteht, wenn er sich bewegt hat. Durch geeigneten Aufbau der Kappe 238 innen und des Verschlusses 210 außen kann verhindert werden, dass der Verschluss 210 aus der Kappe 238 herausfällt. Der Anwender der Vorrichtung kann an dem Herausstehen des Verschlusses 210 aus der Kappe 238 erkennen, dass der Knochenzement in der Kartusche 1 fertig gemischt ist, weil nur dann der Verschluss 210 mittels des fließfähig gewordenen Knochenzements ein Vortreiben des Verschlusses 210 in der Austragsöffnung 9 und in der Kappe 238 ermöglicht. Der Anwender kann dann unmittelbar vor der Anwendung und Verwendung des Knochenzements die Kappe 238 abschrauben und die Kappe 238 mit dem Verschluss 210 darin entfernen.

Der Monomerflüssigkeitsbehälter 8 kann durch Vortreiben des Antriebskolbens 5 aufgebrochen werden und dadurch die Monomerflüssigkeit 7 in dem zweiten Hohlraum 14 freigesetzt werden. Anschließend kann durch weiteres Vortreiben des Antriebskolbens 5 die Monomerflüssigkeit 7 in das Zementpulver 6 im ersten Hohlraum 12 gepresst werden. Durch eine Spannung der Wandungen der Kartusche 1 und durch elastische Verformungen der den zweiten Hohlraum 14 begrenzenden Teile der Vorrichtung steht die Monomerflüssigkeit 7 im zweiten Hohlraum 12 anschließend unter Druck.

Auch die Vorrichtung nach dem dritten Ausführungsbeispiel hat eine Druckentlastungsvorrichtung 216, mit der dieser Druck in dem zweiten Hohlraum 14 durch Ableiten von Teilen der Monomerflüssigkeit 7 aus dem zweiten Hohlraum 12 reduziert werden kann, die sich aber von den Druckentlastungsvorrichtungen 16, 116 des ersten und zweiten Ausführungsbeispiels unterscheidet. Die Druckentlastungsvorrichtung 216 ist an einer seitlichen Oberfläche der Kartusche 1 angeordnet. Theoretisch können auch mehrere gleichartige oder auch unterschiedliche Druckentlastungsvorrichtungen vorgesehen sein, um den Druck der Monomerflüssigkeit 7 in dem zweiten Hohlraum 12 abzubauen. Die Druckentlastungsvorrichtung 216 ist als Querschnitt im Detail in der Ausschnittvergrößerung nach Figur 10 gezeigt.

Die Kartusche 1 weist im Bereich der Druckentlastungsvorrichtung 216 eine durchstechbare dünne Wandung 241 auf, an die sich ein Kanal anschließt, der in ein Reservoir zur Aufnahme von Monomerflüssigkeit 7 in der Druckentlastungsvorrichtung 216 mündet. Hierdurch bildet die Druckentlastungsvorrichtung 216 in ihrem Inneren ein Reservoir zur Aufnahme von Monomerflüssigkeit 7, wobei ein Zellstoff, eine Zellulose oder ein saugfähiges Material (nicht gezeigt) in dem Reservoir angeordnet sein kann, mit dem die Monomerflüssigkeit 7 aufgesaugt und gebunden werden kann.

Zum Durchstechen der dünnen Wandung 241 kann in der Druckentlastungsvorrichtung 216 ein gegen die Kartusche 1 beweglicher Dorn 242 angeordnet sein. Wenn die dünne Wandung 241 mit dem Dorn 242 durchstochen wird, entsteht in der Kartuschenwand der Kartusche 1 eine durchgehende Verbindung durch die Kartuschenwand, die den zweiten Hohlraum 14 für die Monomerflüssigkeit 7 durchlässig mit dem Reservoir in der Druckentlastungsvorrichtung 216 verbindet. Damit der Dorn 242 die durchgehende Verbindung nicht verschließt, kann in dem Dorn 242 eine Furche 254 vorgesehen sein, durch die die Monomerflüssigkeit 7 in das Reservoir einströmen kann.

Theoretisch können auch mehrere dünne Wandungen 241 mit mehreren Dornen 242 vorgesehen sein, die an die gleiche Druckentlastungsvorrichtung 216 oder auch an mehrere gleichartige oder unterschiedliche Druckentlastungsvorrichtungen angeschlossen sind. Der Dorn 242 kann mit einer Federführungsstange 246 mit Hilfe einer Feder 248 von der dünnen Wandung 241 weggedrückt werden. Die Federführungsstange 246 kann innen an einer Kappe 250 der Druckentlastungsvorrichtung 216 angeordnet sein. Die Feder 248 kann unter Druck stehen und sich auf einer Seite außen an der Kartusche 1 und auf der anderen Seite innen an der Kappe 250 abstützen. Die Feder 248 kann um die Federführungsstange 246 herum gewunden sein. Die dünne Wandung 241 kann direkt neben dem Mittelkolben 4 als Wandung des zweiten Hohlraums 14 vorgesehen sein. Dadurch kann sichergestellt werden, dass der Antriebskolben 5 die Einmündung in die mit dem Dorn 242 durchgestochene durchgehende Verbindung nicht verschließt, wenn er in Richtung des Mittelkolbens 4 gedrückt ist.

Die Druckentlastungsvorrichtung 216 kann ferner einen hohlen Stutzen 252 aufweisen, der vorzugsweise einteilig mit der Kartusche 1 ausgeführt ist.

Damit die Monomerflüssigkeit 7 nicht in die Umgebung der Vorrichtung gelangt, kann die Kappe 250 dicht aber gleichzeitig beweglich mit dem Stutzen 252 verbunden sein. Hierzu können an der Kappe 250 Vorsprünge 244 und an dem Stutzen 252 eine Nut 260 vorgesehen sein, wobei die Vorsprünge 244 in die Nut 260 greifen und die Nut 260 so lang ist, dass die Vorsprünge 244 sich in der Nut 260 bewegen können und dass die Kappe 250 sich so weit von der Kartusche 1 abheben kann, dass sich der Dorn 242 die dünne Wandung 241 nicht durchsticht, aber nicht so weit, dass sich die Kappe 250 von dem Stutzen 252 löst oder die Kappe 250 gegen den Stutzen 252 undicht wird. Alternativ könnten auch die Nut 260 in der Kappe 250 und die Vorsprünge 244 an dem Stutzen 252 angeordnet werden. Die Kappe 250 kann manuell gegen die Kraft der Feder 248 gegen die Kartusche 1 gedrückt werden, so dass der Dorn 242 die dünne Wandung 241 durchsticht und dadurch eine durchgehende Verbindung zwischen dem zweiten Hohlraum 14 und dem Reservoir in der Druckentlastungsvorrichtung 216 bereitstellt. Dadurch kann in dem zweiten Hohlraum 14 unter Druck stehende Monomerflüssigkeit 7 in die Druckentlastungsvorrichtung 216 strömen und dadurch der hydrostatische Druck der Monomerflüssigkeit 7 in dem zweiten Hohlraum 14 reduziert werden. Der Anwender kann an dem aus der Kappe 238 hervorstehenden Verschluss 210 erkennen, wann er die Druckentlastung manuell auslösen möchte. Sobald der Verschluss 210 aus der Kappe 238 vorsteht, kann der Anwender also die Druckentlastungsvorrichtung 216 durch einen Druck auf die Kappe 250 auslösen.

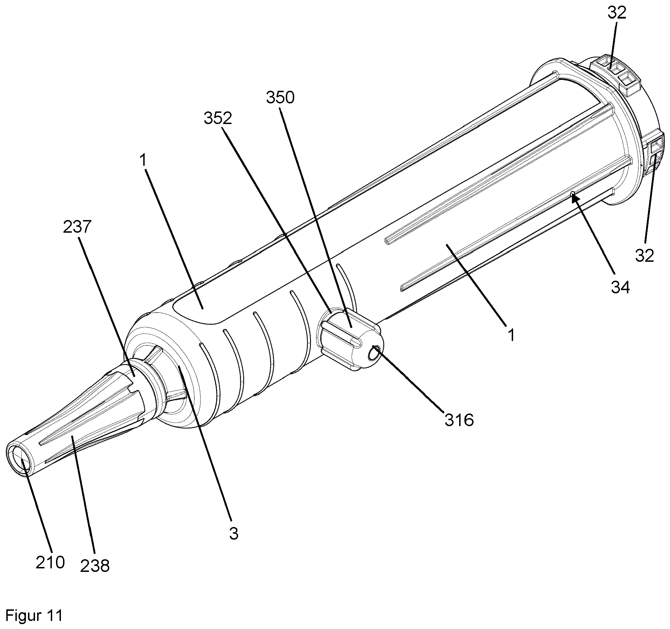

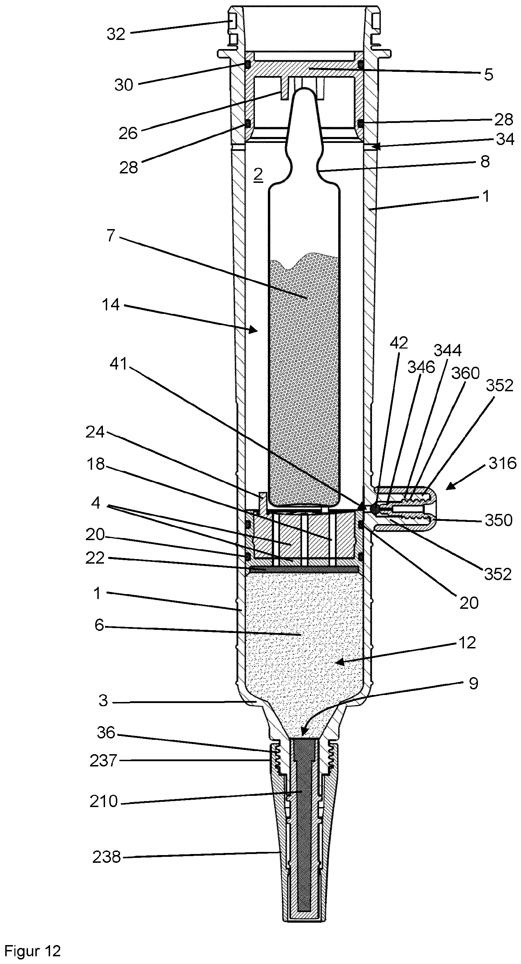

In den Figuren 11 und 12 sind Abbildungen einer vierten erfindungsgemäßen Vorrichtung zum Herstellen eines Knochenzements gezeigt. Die Figuren 11 und 12 zeigen verschiedene schematische Gesamtansichten der vierten beispielhaften erfindungsgemäßen Vorrichtung.

Der Aufbau der vierten erfindungsgemäßen Vorrichtung entspricht in weiten Teilen dem der dritten erfindungsgemäßen Vorrichtung. Die vierte erfindungsgemäße Vorrichtung kann dementsprechend ebenfalls eine Kartusche 1 mit einem zylindrischen Innenraum 2 aufweisen, die an ihrer Vorderseite (in Figur 11 links unten und in Figur 12 unten) mit einem Kartuschenkopf 3 enthaltend eine Austragsöffnung 9 geschlossen ist. Die vierte Vorrichtung weist dementsprechend ebenfalls einen Mittelkolben 4 und einen Antriebskolben 5 auf, die axial in dem zylindrischen Innenraum 2 der Kartusche 3 beweglich angeordnet sind. Auch die Anordnung von Zementpulver 6 und Monomerflüssigkeit 7 in einem Monomerflüssigkeitsbehälter 8 in den von dem Mittelkolben 4 getrennten ersten Hohlraum 12 und zweiten Hohlraum 14 des zylindrischen Innenraums 2 der Kartusche 1 ist analog dem ersten, dem zweiten und dem dritten Ausführungsbeispiel. Der Aufbau des Mittelkolbens 4 mit den Leitungen 18, den beiden Dichtungen 20, dem Porenfilter 22 und der Stifte 24 entspricht genauso dem des ersten und zweiten Ausführungsbeispiels wie der Aufbau des Antriebskolbens 5 mit den Stiften 26, der vorderen Dichtung 28 und der hinteren Dichtung 30. Ebenso können ein Befestigungsmittel 32, Begasungsöffnungen 34 und/oder ein Stutzen mit einem Außengewinde 36 an der Kartusche 1 entsprechend dem ersten und zweiten Ausführungsbeispiel angeordnet sein.

Insofern gleicht das vierte Ausführungsbeispiel dem ersten und dem zweiten Ausführungsbeispiel und für Details zum Aufbau und den Funktionen der entsprechenden Teile des vierten Ausführungsbeispiels wird zur Vermeidung von Wiederholungen auf die Beschreibung zum ersten und zweiten Ausführungsbeispiel verwiesen.

Die Austragsöffnung 9 kann analog dem dritten Ausführungsbeispiel zunächst mit einem Verschluss 210 verschlossen sein, der für Gase durchlässig ist aber für das Zementpulver 6 undurchlässig ist. Eine Kappe 238 kann über eine Schraubverbindung 237 mit dem Außengewinde 36 an der Vorderseite der Kartusche 1 verbunden sein. Der Verschluss 210 ist aus der Austragsöffnung 9 und aus der Kappe 238 herausdrückbar, so dass der Verschluss 210 nach vorne aus der Kappe 238 heraussteht, wenn er sich bewegt hat. Durch geeigneten Aufbau der Kappe 238 innen und des Verschlusses 210 außen kann verhindert werden, dass der Verschluss 210 aus der Kappe 238 herausfällt. Der Anwender der Vorrichtung kann an dem Herausstehen des Verschlusses 210 aus der Kappe 238 erkennen, dass der Knochenzement in der Kartusche 1 fertig gemischt ist, weil nur dann der Verschluss 210 mittels des fließfähig gewordenen Knochenzements ein Vortreiben des Verschlusses 210 in der Austragsöffnung 9 und in der Kappe 238 ermöglicht. Der Anwender kann dann unmittelbar vor der Anwendung und Verwendung des Knochenzements die Kappe 238 abschrauben und die Kappe 238 mit dem Verschluss 210 darin entfernen.

Der Monomerflüssigkeitsbehälter 8 kann durch Vortreiben des Antriebskolbens 5 aufgebrochen werden und dadurch die Monomerflüssigkeit 7 in dem zweiten Hohlraum 14 freigesetzt werden. Anschließend kann durch weiteres Vortreiben des Antriebskolbens 5 die Monomerflüssigkeit 7 in das Zementpulver 6 im ersten Hohlraum 12 gepresst werden. Durch eine Spannung der Wandungen der Kartusche 1 und durch elastische Verformungen der den zweiten Hohlraum 14 begrenzenden Teile der Vorrichtung steht die Monomerflüssigkeit 7 im zweiten Hohlraum 12 anschließend unter Druck.

Auch die Vorrichtung nach dem vierten Ausführungsbeispiel hat eine Druckentlastungsvorrichtung 316, mit der dieser Druck in dem zweiten Hohlraum 14 durch Ableiten von Teilen der Monomerflüssigkeit 7 aus dem zweiten Hohlraum 12 reduziert werden kann, die sich aber von den Druckentlastungsvorrichtungen 16, 116, 216 des ersten, zweiten und dritten Ausführungsbeispiels unterscheidet. Die Druckentlastungsvorrichtung 316 ist an einer seitlichen Oberfläche der Kartusche 1 angeordnet. Theoretisch können auch mehrere gleichartige oder auch unterschiedliche Druckentlastungsvorrichtungen vorgesehen sein, um den Druck der Monomerflüssigkeit 7 in dem zweiten Hohlraum 12 abzubauen.

In der Wandung der Kartusche 1 kann eine durchgehende Verbindung 41 vorgesehen sein, durch die die Druckentlastungsvorrichtung 316 mit dem zweiten Hohlraum 14 für die Monomerflüssigkeit 7 durchlässig verbunden oder verbindbar ist. Theoretisch können auch mehrere durchgehende Verbindungen 41 vorgesehen sein, die an die gleiche Druckentlastungsvorrichtung 316 oder auch an mehrere gleichartige oder unterschiedliche Druckentlastungsvorrichtungen angeschlossen sind. Zum Verschließen der durchgehenden Verbindung 41 kann ein Dichtungskörper 42 auf die Verbindung der Druckentlastungsvorrichtung 316 zur durchgehenden Verbindung 41 gedrückt werden. Der Dichtungskörper 42 kann beispielsweise durch einen Gummipfropfen realisiert werden. Der Dichtungskörper 42 kann mit einer Stange 346 mit Hilfe einer Schraubkappe 350 (beziehungsweise mit Hilfe einer schraubbaren Kappe 350) in den Sitz gepresst werden. Die Stange 346 kann innen an der Schraubkappe 350 der Druckentlastungsvorrichtung 316 angeordnet sein. Die durchgehende Verbindung 41 kann direkt neben dem Mittelkolben 4 in den zweiten Hohlraum 14 münden. Dadurch kann sichergestellt werden, dass der Antriebskolben 5 die Einmündung in die durchgehende Verbindung 41 nicht verschließt, wenn er in Richtung des Mittelkolbens 4 gedrückt ist.

Die Druckentlastungsvorrichtung 316 kann ferner einen hohlen Stutzen 352 aufweisen, der vorzugsweise einteilig mit der Kartusche 1 ausgeführt ist. Die Schraubkappe 350 weist ein Außengewinde 344 auf und der Stutzen 352 ein dazu passendes Innengewinde 360. Dadurch kann die Schraubkappe 350 von dem Stutzten 352 weggeschraubt, aber vorzugsweise nicht gelöst werden. Dadurch löst sich der Dichtungskörper 42 von der durchgehenden Verbindung 41. Dadurch kann in dem zweiten Hohlraum 14 unter Druck stehende Monomerflüssigkeit 7 in die Druckentlastungsvorrichtung 316 strömen und dadurch der hydrostatische Druck der Monomerflüssigkeit 7 in dem zweiten Hohlraum 14 reduziert werden.

Damit die Monomerflüssigkeit 7 nicht in die Umgebung der Vorrichtung gelangt, kann die Schraubkappe 350 dicht aber gleichzeitig beweglich mit dem Stutzen 352 verbunden sein. Hierdurch bildet die Druckentlastungsvorrichtung 316 in ihrem Inneren ein Reservoir zur Aufnahme von Monomerflüssigkeit 7, wobei ein Zellstoff, eine Zellulose oder ein saugfähiges Material (nicht gezeigt) in dem Reservoir angeordnet sein kann, mit dem die Monomerflüssigkeit 7 aufgesaugt und gebunden werden kann.

Der Anwender kann an dem aus der Kappe 238 hervorstehenden Verschluss 210 erkennen, wann er die Druckentlastung manuell auslösen möchte. Sobald der Verschluss 210 aus der Kappe 238 vorsteht, kann der Anwender also die Druckentlastungsvorrichtung 316 durch Abschrauben beziehungsweise durch eine Drehung zum Lösen der Schraubkappe 350 und damit zum Lösen des Dichtungskörpers 42 auslösen.

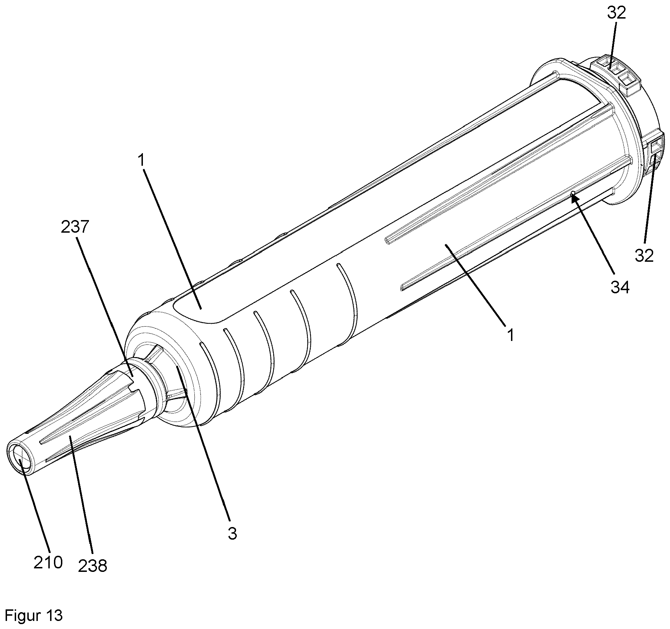

In den Figuren 13 bis 15 sind Abbildungen einer fünften erfindungsgemäßen Vorrichtung zum Herstellen eines Knochenzements gezeigt. Die Figuren 13 bis 15 zeigen verschiedene schematische Gesamtansichten der fünften beispielhaften erfindungsgemäßen Vorrichtung.

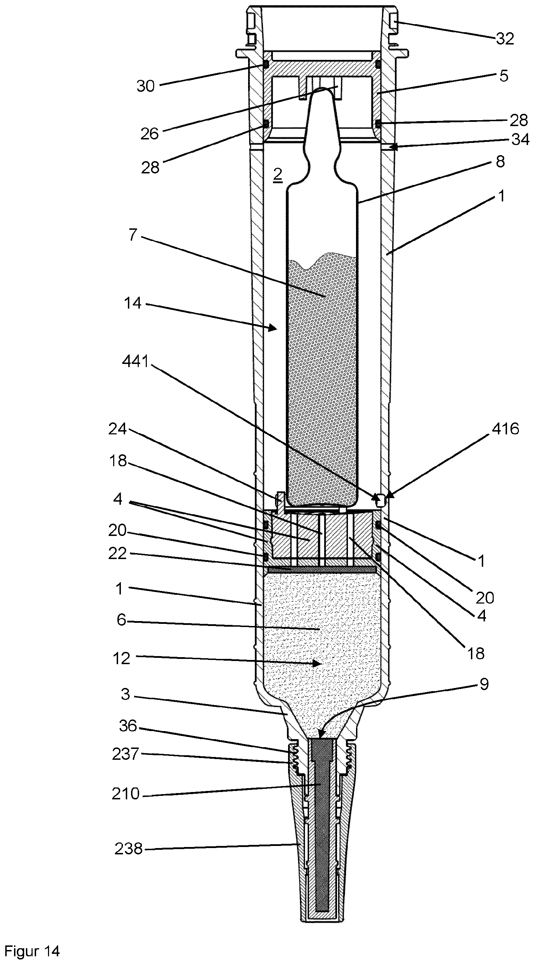

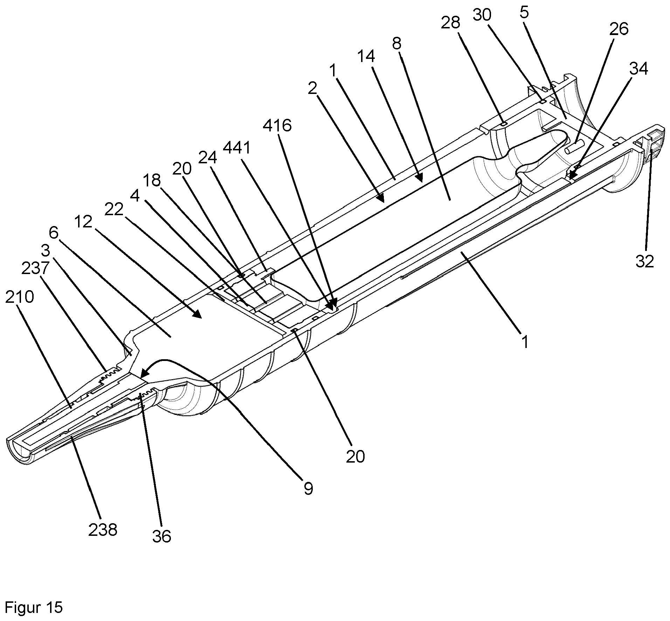

Der Aufbau der fünften erfindungsgemäßen Vorrichtung entspricht in weiten Teilen dem der ersten und der zweiten erfindungsgemäßen Vorrichtung sowie der dritten und vierten erfindungsgemäßen Vorrichtung. Die fünfte erfindungsgemäße Vorrichtung kann dementsprechend ebenfalls eine Kartusche 1 mit einem zylindrischen Innenraum 2 aufweisen, die an ihrer Vorderseite (in den Figuren 13 und 15 links unten und in Figur 14 unten) mit einem Kartuschenkopf 3 enthaltend eine Austragsöffnung 9 geschlossen ist. Die fünfte Vorrichtung weist dementsprechend ebenfalls einen Mittelkolben 4 und einen Antriebskolben 5 auf, die axial in dem zylindrischen Innenraum 2 der Kartusche 3 beweglich angeordnet sind. Auch die Anordnung von Zementpulver 6 und Monomerflüssigkeit 7 in einem Monomerflüssigkeitsbehälter 8 in den von dem Mittelkolben 4 getrennten ersten Hohlraum 12 und zweiten Hohlraum 14 des zylindrischen Innenraums 2 der Kartusche 1 ist analog dem ersten und dem zweiten Ausführungsbeispiel. Der Aufbau des Mittelkolbens 4 mit den Leitungen 18, den beiden Dichtungen 20, dem Porenfilter 22 und der Stifte 24 entspricht genauso dem des ersten und zweiten Ausführungsbeispiels wie der Aufbau des Antriebskolbens 5 mit den Stiften 26, der vorderen Dichtung 28 und der hinteren Dichtung 30. Ebenso können ein Befestigungsmittel 32, Begasungsöffnungen 34 und/oder ein Stutzen mit einem Außengewinde 36 an der Kartusche 1 entsprechend dem ersten und zweiten Ausführungsbeispiel angeordnet sein.

Insofern gleicht das fünfte Ausführungsbeispiel dem ersten und dem zweiten Ausführungsbeispiel und für Details zum Aufbau und den Funktionen der entsprechenden Teile des dritten Ausführungsbeispiels wird zur Vermeidung von Wiederholungen auf die Beschreibung zum ersten und zweiten Ausführungsbeispiel verwiesen.

Die Austragsöffnung 9 kann analog dem dritten und vierten Ausführungsbeispiel zunächst mit einem Verschluss 210 verschlossen sein, der für Gase durchlässig ist aber für das Zementpulver 6 undurchlässig ist. Eine Kappe 238 kann über eine Schraubverbindung 237 mit dem Außengewinde 36 an der Vorderseite der Kartusche 1 verbunden sein. Der Verschluss 210 ist aus der Austragsöffnung 9 und aus der Kappe 238 herausdrückbar, so dass der Verschluss 210 nach vorne aus der Kappe 238 heraussteht, wenn er sich bewegt hat. Durch geeigneten Aufbau der Kappe 238 innen und des Verschlusses 210 außen kann verhindert werden, dass der Verschluss 210 aus der Kappe 238 herausfällt. Der Anwender der Vorrichtung kann an dem Herausstehen des Verschlusses 210 aus der Kappe 238 erkennen, dass der Knochenzement in der Kartusche 1 fertig gemischt ist, weil nur dann der Verschluss 210 mittels des fließfähig gewordenen Knochenzements ein Vortreiben des Verschlusses 210 in der Austragsöffnung 9 und in der Kappe 238 ermöglicht. Der Anwender kann dann unmittelbar vor der Anwendung und Verwendung des Knochenzements die Kappe 238 abschrauben und die Kappe 238 mit dem Verschluss 210 darin entfernen.

Der Monomerflüssigkeitsbehälter 8 kann durch Vortreiben des Antriebskolbens 5 aufgebrochen werden und dadurch die Monomerflüssigkeit 7 in dem zweiten Hohlraum 14 freigesetzt werden. Anschließend kann durch weiteres Vortreiben des Antriebskolbens 5 die Monomerflüssigkeit 7 in das Zementpulver 6 im ersten Hohlraum 12 gepresst werden. Durch eine Spannung der Wandungen der Kartusche 1 und durch elastische Verformungen der den zweiten Hohlraum 14 begrenzenden Teile der Vorrichtung steht die Monomerflüssigkeit 7 im zweiten Hohlraum 12 anschließend unter Druck.

Auch die Vorrichtung nach dem fünften Ausführungsbeispiel hat eine Druckentlastungsvorrichtung 416, mit der dieser Druck in dem zweiten Hohlraum 14 durch Ableiten von Teilen der Monomerflüssigkeit 7 aus dem zweiten Hohlraum 12 reduziert werden kann, die sich aber von den Druckentlastungsvorrichtungen 16, 116, 216, 316 des ersten, zweiten, dritten und vierten Ausführungsbeispiels unterscheidet. Die Druckentlastungsvorrichtung 416 ist an einer seitlichen inneren Oberfläche der Kartusche 1 angeordnet. Theoretisch können auch mehrere gleichartige oder auch unterschiedliche Druckentlastungsvorrichtungen vorgesehen sein, um den Druck der Monomerflüssigkeit 7 in dem zweiten Hohlraum 12 abzubauen.

Die Kartusche 1 weist im Bereich der Druckentlastungsvorrichtung 416 eine Nut 441 auf, über die die vordere Dichtung 28 des Antriebskolbens 5 geschoben wird, wenn der Antriebskolben 5 weit genug in Richtung des Mittelkolbens 4 gedrückt wird. Dadurch entsteht eine für die Monomerflüssigkeit durchlässige Verbindung zwischen dem zweiten Hohlraum 14 und einem Reservoir zwischen der Außenseite des Antriebskolbens 5 und der Innenwand der Kartusche 1, der auf seiner Rückseite durch die zweite Dichtung 30 des Antriebskolbens 5 abgedichtet ist. Der Antriebskolben 5 kann an seiner Mantelfläche, die der Innenwand der Kartusche 1 zugewandt ist, zumindest eine Vertiefung zur Aufnahme von Monomerflüssigkeit 7 aufweisen. Dadurch kann das Reservoir vergrößert werden. Das Reservoir ist zur Aufnahme von Monomerflüssigkeit 7 vorgesehen, wobei ein Zellstoff, eine Zellulose oder ein saugfähiges Material (nicht gezeigt) in dem Reservoir angeordnet sein kann, mit dem die Monomerflüssigkeit 7 aufgesaugt und gebunden werden kann. Das Reservoir wird automatisch durch Überfahren der Nut 441 mit der vorderen Dichtung 28 geöffnet und dadurch eine automatische Druckentlastung des hydrostatischen Drucks der Monomerflüssigkeit 7 in dem zweiten Hohlraum 14 erreicht.

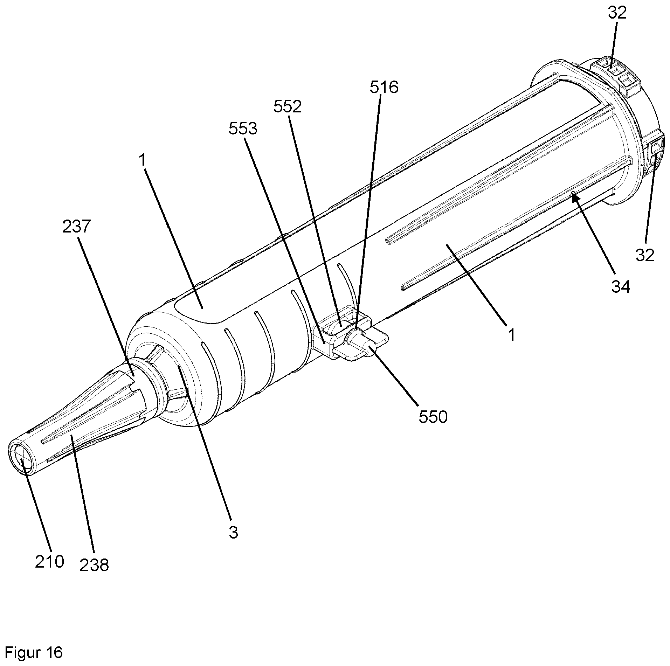

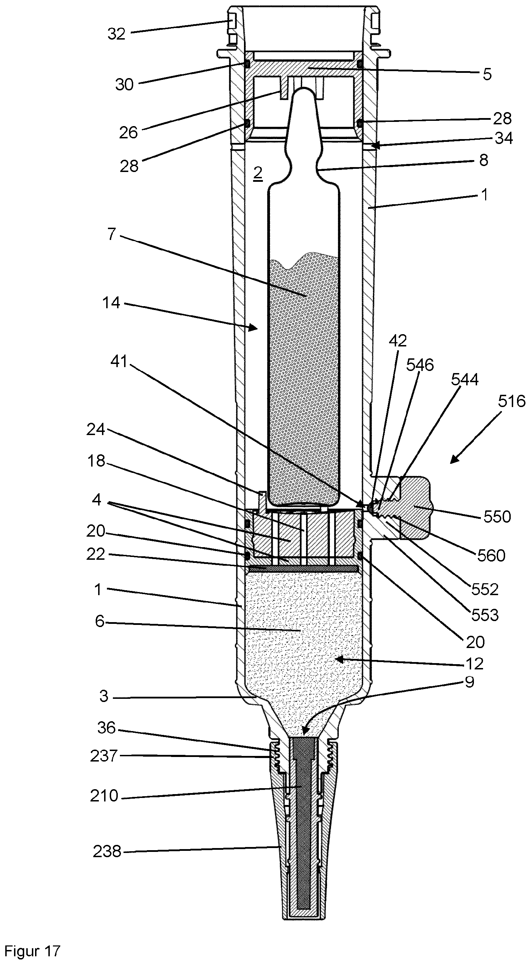

In den Figuren 16 und 17 sind Abbildungen einer sechsten erfindungsgemäßen Vorrichtung zum Herstellen eines Knochenzements gezeigt, die der vierten erfindungsgemäßen Vorrichtung sehr ähnlich ist. Die Figuren 16 und 17 zeigen verschiedene schematische Gesamtansichten der vierten beispielhaften erfindungsgemäßen Vorrichtung.

Der Aufbau der sechsten erfindungsgemäßen Vorrichtung entspricht dementsprechend in weiten Teilen dem der vierten erfindungsgemäßen Vorrichtung. Die sechste erfindungsgemäße Vorrichtung kann dementsprechend ebenfalls eine Kartusche 1 mit einem zylindrischen Innenraum 2 aufweisen, die an ihrer Vorderseite (in Figur 16 links unten und in Figur 17 unten) mit einem Kartuschenkopf 3 enthaltend eine Austragsöffnung 9 geschlossen ist. Die sechste Vorrichtung weist dementsprechend ebenfalls einen Mittelkolben 4 und einen Antriebskolben 5 auf, die axial in dem zylindrischen Innenraum 2 der Kartusche 3 beweglich angeordnet sind. Auch die Anordnung von Zementpulver 6 und Monomerflüssigkeit 7 in einem Monomerflüssigkeitsbehälter 8 in den von dem Mittelkolben 4 getrennten ersten Hohlraum 12 und zweiten Hohlraum 14 des zylindrischen Innenraums 2 der Kartusche 1 ist analog den anderen Ausführungsbeispielen. Der Aufbau des Mittelkolbens 4 mit den Leitungen 18, den beiden Dichtungen 20, dem Porenfilter 22 und der Stifte 24 entspricht genauso dem des ersten und zweiten Ausführungsbeispiels wie der Aufbau des Antriebskolbens 5 mit den Stiften 26, der vorderen Dichtung 28 und der hinteren Dichtung 30. Ebenso können ein Befestigungsmittel 32, Begasungsöffnungen 34 und/oder ein Stutzen mit einem Außengewinde 36 an der Kartusche 1 entsprechend dem ersten und zweiten Ausführungsbeispiel angeordnet sein.

Insofern gleicht das sechste Ausführungsbeispiel dem ersten und dem zweiten Ausführungsbeispiel und für Details zum Aufbau und den Funktionen der entsprechenden Teile des sechsten Ausführungsbeispiels wird zur Vermeidung von Wiederholungen auf die Beschreibung zum ersten und zweiten Ausführungsbeispiel verwiesen.

Die Austragsöffnung 9 kann analog dem vierten Ausführungsbeispiel zunächst mit einem Verschluss 210 verschlossen sein, der für Gase durchlässig ist aber für das Zementpulver 6 undurchlässig ist. Eine Kappe 238 kann über eine Schraubverbindung 237 mit dem Außengewinde 36 an der Vorderseite der Kartusche 1 verbunden sein. Der Verschluss 210 ist aus der Austragsöffnung 9 und aus der Kappe 238 herausdrückbar, so dass der Verschluss 210 nach vorne aus der Kappe 238 heraussteht, wenn er sich bewegt hat. Durch geeigneten Aufbau der Kappe 238 innen und des Verschlusses 210 außen kann verhindert werden, dass der Verschluss 210 aus der Kappe 238 herausfällt. Der Anwender der Vorrichtung kann an dem Herausstehen des Verschlusses 210 aus der Kappe 238 erkennen, dass der Knochenzement in der Kartusche 1 fertig gemischt ist, weil nur dann der Verschluss 210 mittels des fließfähig gewordenen Knochenzements ein Vortreiben des Verschlusses 210 in der Austragsöffnung 9 und in der Kappe 238 ermöglicht. Der Anwender kann dann unmittelbar vor der Anwendung und Verwendung des Knochenzements die Kappe 238 abschrauben und die Kappe 238 mit dem Verschluss 210 darin entfernen.

Der Monomerflüssigkeitsbehälter 8 kann durch Vortreiben des Antriebskolbens 5 aufgebrochen werden und dadurch die Monomerflüssigkeit 7 in dem zweiten Hohlraum 14 freigesetzt werden. Anschließend kann durch weiteres Vortreiben des Antriebskolbens 5 die Monomerflüssigkeit 7 in das Zementpulver 6 im ersten Hohlraum 12 gepresst werden. Durch eine Spannung der Wandungen der Kartusche 1 und durch elastische Verformungen der den zweiten Hohlraum 14 begrenzenden Teile der Vorrichtung steht die Monomerflüssigkeit 7 im zweiten Hohlraum 12 anschließend unter Druck.

Auch die Vorrichtung nach dem sechsten Ausführungsbeispiel hat eine Druckentlastungsvorrichtung 516, mit der dieser Druck in dem zweiten Hohlraum 14 durch Ableiten von Teilen der Monomerflüssigkeit 7 aus dem zweiten Hohlraum 12 reduziert werden kann, die sich aber von den Druckentlastungsvorrichtungen 16, 116, 216, 316, 416 der anderen Ausführungsbeispiele unterscheidet, allerdings der Druckentlastungsvorrichtung 316 des vierten Ausführungsbeispiels sehr ähnlich in der Funktion ist. Die Druckentlastungsvorrichtung 516 ist an einer seitlichen Oberfläche der Kartusche 1 angeordnet. Theoretisch können auch mehrere gleichartige oder auch unterschiedliche Druckentlastungsvorrichtungen vorgesehen sein, um den Druck der Monomerflüssigkeit 7 in dem zweiten Hohlraum 12 abzubauen.

In der Wandung der Kartusche 1 kann eine durchgehende Verbindung 41 vorgesehen sein, durch die die Druckentlastungsvorrichtung 516 mit dem zweiten Hohlraum 14 für die Monomerflüssigkeit 7 durchlässig verbunden oder verbindbar ist. Theoretisch können auch mehrere durchgehende Verbindungen 41 vorgesehen sein, die an die gleiche Druckentlastungsvorrichtung 516 oder auch an mehrere gleichartige oder unterschiedliche Druckentlastungsvorrichtungen angeschlossen sind. Zum Verschließen der durchgehenden Verbindung 41 kann ein Dichtungskörper 42 auf die Verbindung der Druckentlastungsvorrichtung 516 zur durchgehenden Verbindung 41 gedrückt werden. Der Dichtungskörper 42 kann beispielsweise durch einen Gummipfropfen realisiert werden. Der Dichtungskörper 42 kann mit einer Stange 546 mit Hilfe einer Schraube 550 in den Sitz gepresst werden. Die Stange 546 ist die Spitze der Schraube 550. Die durchgehende Verbindung 41 kann direkt neben dem Mittelkolben 4 in den zweiten Hohlraum 14 münden. Dadurch kann sichergestellt werden, dass der Antriebskolben 5 die Einmündung in die durchgehende Verbindung 41 nicht verschließt, wenn er in Richtung des Mittelkolbens 4 gedrückt ist.

Die Druckentlastungsvorrichtung 516 kann ferner einen hohlen Stutzen 552 mit einer Verstrebung 553 zur mechanischen Stabilisierung aufweisen, die vorzugsweise einteilig mit der Kartusche 1 ausgeführt sind. Die Schraube 550 weist ein Außengewinde 544 auf und der Stutzen 552 ein dazu passendes Innengewinde 560. Dadurch kann die Schraube 550 in dem Stutzten 552 geschraubt, aber vorzugsweise nicht getrennt werden. Durch das Lösen der Schraube 550 löst sich der Dichtungskörper 42 von der durchgehenden Verbindung 41. Dadurch kann in dem zweiten Hohlraum 14 unter Druck stehende Monomerflüssigkeit 7 in die Druckentlastungsvorrichtung 516 strömen und dadurch der hydrostatische Druck der Monomerflüssigkeit 7 in dem zweiten Hohlraum 14 reduziert werden.

Damit die Monomerflüssigkeit 7 nicht in die Umgebung der Vorrichtung gelangt, kann die Verbindung der Schraube 550 mit dem Stutzen 352 dicht sein. Hierdurch bildet die Druckentlastungsvorrichtung 516 in ihrem Inneren ein Reservoir zur Aufnahme von Monomerflüssigkeit 7, wobei ein Zellstoff, eine Zellulose oder ein saugfähiges Material (nicht gezeigt) in dem Reservoir angeordnet sein kann, mit dem die Monomerflüssigkeit 7 aufgesaugt und gebunden werden kann.

Der Anwender kann an dem aus der Kappe 238 hervorstehenden Verschluss 210 erkennen, wann er die Druckentlastung manuell auslösen möchte. Sobald der Verschluss 210 aus der Kappe 238 vorsteht, kann der Anwender also die Druckentlastungsvorrichtung 516 durch Abschrauben beziehungsweise durch eine Drehung zum Lösen der Schraube 550 und damit zum Lösen des Dichtungskörpers 42 auslösen.

### Bezugszeichenliste

- 1: Kartusche
- 2: Innenraum
- 3: Kartuschenkopf
- 4: Mittelkolben
- 5: Antriebskolben
- 6: Zementpulver
- 7: Monomerflüssigkeit
- 8: Monomerflüssigkeitsbehälter
- 9: Austragsöffnung
- 10,210: Verschluss
- 12: erster Hohlraum
- 14: zweiter Hohlraum
- 16, 116, 216, 316, 416, 516: Druckentlastungsvorrichtung
- 18: Leitung
- 20: Dichtung
- 22: Porenfilter
- 24, 26: Stift
- 28: vordere Dichtung
- 30: hintere Dichtung
- 32: Befestigungsmittel
- 34: Begasungsöffnung
- 36: Außengewinde
- 38, 138: Verbindungselement
- 40: Zapfen
- 41: Verbindung
- 42: Dichtungskörper
- 44, 144, 244: Vorsprung
- 46, 146, 246: Federführungsstange
- 48, 148, 248: Feder
- 50,150,250: Kappe
- 52, 152, 252, 352, 552: Stutzen
- 54: Durchgang
- 56: Führung
- 58: Stopper
- 60, 160, 260: Nut
- 140: Stützkörper
- 156: Schiene
- 237: Schraubverbindung
- 238: Kappe
- 241: dünne Wandung
- 242: Dorn
- 254: Furche
- 344: Außengewinde
- 346: Stange
- 350: Schraubkappe
- 360: Innengewinde
- 441: Nut
- 544: Außengewinde
- 546: Stange
- 550: Schraube
- 553: Verstrebung
- 560: Innengewinde

## Patentansprüche

1. Verfahren zur Herstellung eines Knochenzements, insbesondere eines pastenförmigen Polymethylmethacrylat-Knochenzements, wobei der Knochenzement aus einem Zementpulver (6) und einer Monomerflüssigkeit (7) mit einer Vorrichtung zum Mischen des Knochenzements hergestellt wird, die Vorrichtung aufweisend
eine Kartusche (1) mit einem zylindrischen Innenraum (2),
ein Zementpulver (6) zur Herstellung des Knochenzements,
eine Monomerflüssigkeit (7) zur Herstellung des Knochenzements, wobei die Monomerflüssigkeit (7) in einem Monomerflüssigkeitsbehälter (8) enthalten ist,
einen Kartuschenkopf (3) mit einer Austragsöffnung (9) zum Austreiben des Knochenzements, wobei der Kartuschenkopf (3) den zylindrischen Innenraum (2) der Kartusche (1) an einer Vorderseite der Kartusche (1) bis auf die Austragsöffnung (9) verschließt,
einen Verschluss (10, 210), wobei der Verschluss (10, 210) für Gase durchlässig und für Pulverpartikel des Zementpulvers (6) undurchlässig ist und wobei der Verschluss (10, 210) in der Austragsöffnung (9) angeordnet ist und aus der Austragsöffnung (9) entfernbar ist,
einen Antriebskolben (5), wobei der Antriebskolben (5) für Gase und die Monomerflüssigkeit (7) undurchlässig ist, wobei der Antriebskolben (5) in Richtung des Kartuschenkopfs (3) beweglich im zylindrischen Innenraum (2) der Kartusche (1) angeordnet ist,
einen Mittelkolben (4), wobei der Mittelkolben (4) für Gase und die Monomerflüssigkeit (7) durchlässig und für die Pulverpartikel des Zementpulvers (6) undurchlässig ist, wobei der Mittelkolben (4) in Richtung des Kartuschenkopfs (3) beweglich im zylindrischen Innenraum (2) der Kartusche (1) angeordnet ist und zwischen dem Antriebskolben (5) und dem Kartuschenkopf (3) angeordnet ist, wobei der Mittelkolben (4) den zylindrischen Innenraum (2) der Kartusche (1) in einen ersten Hohlraum (12) und einen zweiten Hohlraum (14) trennt, wobei der erste Hohlraum (12) an einer Vorderseite durch den Kartuschenkopf (3) und den Verschluss (10, 210), gegenüberliegend durch den Mittelkolben (4) und seitlich durch eine Innenwand der Kartusche (1) begrenzt ist und wobei der zweite Hohlraum (14) an einer Vorderseite durch den Mittelkolben (4), gegenüberliegend durch den Antriebskolben (5) und seitlich durch die Innenwand der Kartusche (1) begrenzt ist, wobei das Zementpulver (6) in dem ersten Hohlraum (12) angeordnet ist und wobei der Monomerflüssigkeitsbehälter (8) in dem zweiten Hohlraum (14) angeordnet ist,
wobei das Verfahren die folgenden Schritte aufweist:
A) Bewegen des Antriebskolbens (5) in Richtung des Kartuschenkopfs (3),
B) Öffnen des Monomerflüssigkeitsbehälters (8) oder Zerbrechen des Monomerflüssigkeitsbehälters (8) durch die Bewegung des Antriebskolbens (5) in Richtung Kartuschenkopf (3) und dadurch Freisetzen der Monomerflüssigkeit (7) in dem zweiten Hohlraum (14),
C) Auspressen überstehender Gase durch den Mittelkolben (4), durch das Zementpulver (6) und durch den Verschluss (10, 210) hindurch in die Umgebung der Vorrichtung mittels der Bewegung des Antriebskolbens (5),
D) Einpressen der Monomerflüssigkeit (7) durch den Mittelkolben (4) in das Zementpulver (6) durch weiteres Bewegen des Antriebskolbens (5) in Richtung Kartuschenkopf (3),
E) Verdrängen von Gasen zwischen den Pulverpartikeln mit der einfließenden Monomerflüssigkeit (7), wobei die Gase durch den Verschluss (10, 210) in die Umgebung der Vorrichtung entweichen,
F) Benetzen der Pulverpartikel des Zementpulvers (6),
**gekennzeichnet durch** den weiteren Verfahrensschritt:
G) Druckentlasten des zweiten Hohlraums (14) nach den Schritten A) bis F), wobei das Druckentlasten des zweiten Hohlraums (14) durch ein teilweises Entfernen der in dem zweiten Hohlraum (14) enthaltenen Monomerflüssigkeit (7) erfolgt.

2. Verfahren nach Anspruch 1, **dadurch gekennzeichnet, dass**
der Druck in dem zweiten Hohlraum (14) durch die Druckentlastung zumindest um 30% reduziert wird, besonders bevorzugt um zumindest 60% reduziert wird, ganz besonders bevorzugt um zumindest 90% reduziert wird.

3. Verfahren nach Anspruch 1 oder 2, **dadurch gekennzeichnet, dass**
das Bewegen des Antriebskolbens (5) in Richtung des Kartuschenkopfs (3) durch eine äußere Auspressvorrichtung angetrieben wird, wobei bevorzugt die Vorrichtung zum Mischen des Knochenzements in die Auspressvorrichtung eingesetzt wird und/oder an der Auspressvorrichtung befestigt wird.

4. Verfahren nach einem der vorangehenden Ansprüche, **dadurch gekennzeichnet, dass** in Schritt F) die Pulverpartikel des Zementpulvers (6) vollständig von der Monomerflüssigkeit (7) benetzt werden.

5. Verfahren nach einem der vorangehenden Ansprüche, **gekennzeichnet durch** die chronologischen Schritte
H) Entfernen des Verschlusses (10, 210) aus dem Kartuschenkopf (3) nach Schritt G) und
I) Auspressen des Knochenzements aus dem ersten Hohlraum (12) und durch die Austragsöffnung (9) durch eine Bewegung des Mittelkolbens (4) in Richtung des Kartuschenkopfs (3), wobei der Mittelkolben (4) von dem Antriebskolben (5) in Richtung des Kartuschenkopfs (3) gedrückt wird.

6. Verfahren nach einem der vorangehenden Ansprüche, **dadurch gekennzeichnet, dass**
das Verfahren nicht zur medizinischen, diagnostischen oder therapeutischen Behandlung eines menschlichen oder tierischen Körpers angewendet wird.

7. Verfahren nach einem der vorangehenden Ansprüche, **dadurch gekennzeichnet, dass**
das Druckentlasten in Schritt G) durch eine manuelle oder eine automatische Betätigung einer Druckentlastungsvorrichtung (16, 116, 216, 316, 416, 516) der Vorrichtung zum Mischen des Knochenzements erfolgt, wobei bevorzugt die automatische Betätigung der Druckentlastungsvorrichtung (16, 116, 216, 316, 416, 516) durch eine Bewegung des Antriebskolbens (5) ausgelöst wird und/oder durch eine Bewegung des Verschlusses (10, 210) aus dem Kartuschenkopf (3) ausgelöst wird und besonders bevorzugt durch die Bewegung des Verschlusses (10, 210) aus dem Kartuschenkopf (3) angetrieben wird, und/oder
bei dem Druckentlasten in Schritt G) die Monomerflüssigkeit (7) in ein Reservoir außerhalb der Kartusche (1) oder in der Kartuschenwand abgeleitet wird, wobei bevorzugt das Reservoir nach außen für die Monomerflüssigkeit (7) dicht geschlossen ist, oder
bei dem Druckentlasten in Schritt G) die Monomerflüssigkeit (7) in ein Reservoir innerhalb des Antriebskolbens (5), in ein Reservoir auf der dem zweiten Hohlraum (14) gegenüberliegenden Seite des Antriebskolbens (5) und/oder in ein Reservoir seitlich zwischen dem Antriebskolben (5) und der Kartusche (1) geleitet wird, wobei bevorzugt in letzterem Fall die Monomerflüssigkeit (7) durch zumindest eine Nut (441) in einer Innenwand der Kartusche (1) an einem vorderen Dichtungsring (28) des Antriebskolbens (5) vorbei in das Reservoir seitlich zwischen dem Antriebskolben (5) und der Kartusche (1) geleitet wird, wenn ein vorderer Dichtungsring (28) des Antriebskolbens (5) über oder auf die zumindest eine Nut (441) geschoben wird.

8. Verfahren nach einem der vorangehenden Ansprüche, **dadurch gekennzeichnet, dass**
bei dem Druckentlasten in Schritt G) die Monomerflüssigkeit (7) nicht in den ersten Hohlraum (12) geleitet wird, bevorzugt in zumindest ein zum ersten Hohlraum (12) separates Reservoir geleitet wird, wobei besonders bevorzugt das zumindest eine Reservoir außerhalb der Kartusche (1), in der Kartuschenwand, innerhalb des Antriebskolbens (5), auf der dem zweiten Hohlraum (14) gegenüberliegenden Seite
des Antriebskolbens (5) und/oder seitlich zwischen dem Antriebskolben (5) und der Kartusche (1) angeordnet ist, und/oder
bei dem Druckentlasten in Schritt G) der zweite Hohlraum (14) mit dem geöffneten oder zerbrochenen Monomerflüssigkeitsbehälter (8) und mit Resten der Monomerflüssigkeit (7) gefüllt ist.

9. Vorrichtung zum Mischen eines Knochenzements, die Vorrichtung aufweisend
eine Kartusche (1) mit einem zylindrischen Innenraum (2),
ein Zementpulver (6) zur Herstellung des Knochenzements,
eine Monomerflüssigkeit (7) zur Herstellung des Knochenzements, wobei die Monomerflüssigkeit (7) in einem Monomerflüssigkeitsbehälter (8) enthalten ist, einen Kartuschenkopf (3) mit einer Austragsöffnung (9) zum Austreiben des Knochenzements, wobei der Kartuschenkopf (3) den zylindrischen Innenraum (2) der Kartusche (1) an einer Vorderseite der Kartusche (1) bis auf die Austragsöffnung (9) verschließt,
einen Verschluss (10, 210), wobei der Verschluss (10, 210) für Gase durchlässig und für Pulverpartikel des Zementpulvers (6) undurchlässig ist und wobei der Verschluss (10, 210) in der Austragsöffnung (9) angeordnet ist und aus der Austragsöffnung (9) entfernbar ist,
einen Antriebskolben (5), wobei der Antriebskolben (5) für Gase und die Monomerflüssigkeit (7) undurchlässig ist, wobei der Antriebskolben (5) in Richtung des Kartuschenkopfs (3) beweglich im zylindrischen Innenraum (2) der Kartusche (1) angeordnet ist,
einen Mittelkolben (4), wobei der Mittelkolben (4) für Gase und die Monomerflüssigkeit (7) durchlässig und für die Pulverpartikel des Zementpulvers (6) undurchlässig ist, wobei der Mittelkolben (4) in Richtung des Kartuschenkopfs (3) beweglich im zylindrischen Innenraum (2) der Kartusche (1) angeordnet ist und zwischen dem Antriebskolben (5) und dem Kartuschenkopf (3) angeordnet ist, wobei der Mittelkolben (4) den zylindrischen Innenraum (2) der Kartusche (1) in einen ersten Hohlraum (12) und einen zweiten Hohlraum (14) trennt, wobei der erste Hohlraum (12) an einer Vorderseite durch den Kartuschenkopf (3) und den Verschluss (10, 210), gegenüberliegend durch den Mittelkolben (4) und seitlich durch eine Innenwand der Kartusche (1) begrenzt ist und wobei der zweite Hohlraum (14) an einer Vorderseite durch den Mittelkolben (4),
gegenüberliegend durch den Antriebskolben (5) und seitlich durch die Innenwand der Kartusche (1) begrenzt ist, wobei das Zementpulver (6) in dem ersten Hohlraum (12) angeordnet ist und wobei der Monomerflüssigkeitsbehälter (8) in dem zweiten Hohlraum (14) angeordnet ist,
**dadurch gekennzeichnet, dass** die Vorrichtung weiter aufweist:
eine Druckentlastungsvorrichtung (16, 116, 216, 316, 416, 516), mit der Monomerflüssigkeit (7) aus dem zweiten Hohlraum (14) ableitbar oder ableitbar und aufnehmbar ist oder mit der das Volumen des zweiten Hohlraums (14) vergrößerbar ist, wobei die Druckentlastungsvorrichtung (16, 116, 216, 316, 416, 516) mit dem zweiten Hohlraum (14) verbunden oder verbindbar ist.

10. Vorrichtung nach Anspruch 9, **dadurch gekennzeichnet, dass**
die Vorrichtung zum Umsetzen eines Verfahrens nach einem der Ansprüche 1 bis 8 geeignet ist.

11. Vorrichtung nach Anspruch 9 oder 10, **dadurch gekennzeichnet, dass**
die Druckentlastungsvorrichtung (16, 116, 216, 316, 416, 516) über mindestens eine durchgehende Verbindung (41) in einer Wandung der Kartusche (1) mit dem zweiten Hohlraum (14) verbunden ist, wobei die mindestens eine durchgehende Verbindung (41) mit mindestens einem Dichtungskörper (42) verschlossen oder verschließbar ist, wobei bevorzugt der mindestens eine Dichtungskörper (42) mit einer von außen manuell bedienbaren Schraube (550) oder Schraubkappe (350), mit einer Stange (346) oder mit einer Federführungsstange (46, 146, 246) gegen die mindestens eine durchgehende Verbindung (41) gedrückt ist.

12. Vorrichtung nach Anspruch 11, **dadurch gekennzeichnet, dass**
der mindestens eine Dichtungskörper (42) oder eine auf den mindestens einen Dichtungskörper (42) drückende Stange (346) oder Federführungsstange (46, 146, 246) mit einer Feder (48, 148, 248) von der mindestens eine durchgehende Verbindung (41) abhebbar ist, bevorzugt die Feder (48, 148, 248) die Federführungsstange 46, 146, 246), die den mindestens einen Dichtungskörper (42) gegen die mindestens eine durchgehende Verbindung (41) drückt, von der mindestens einen durchgehenden Verbindung (41) weg drückt, wobei besonders bevorzugt die Feder (48, 148) mit einem Zapfen (40) oder mit einem gabelförmigen Stützkörper (140) arretiert ist und der Zapfen (40) oder der gabelförmige Stützkörper (140) mit dem Verschluss (10) fest verbunden ist, so dass bei einer Bewegung des Verschlusses (10) aus der Austragsöffnung (9) auch der Zapfen (40) oder der gabelförmige Stützkörper (140) automatisch aus der Druckentlastungsvorrichtung (16, 116) entfernt wird und dadurch die Arretierung der Feder (48, 148) gelöst wird.

13. Vorrichtung nach Anspruch 9 oder 10, **dadurch gekennzeichnet, dass**
mindestens eine durchgehende Verbindung (41) in der Kartuschenwand angeordnet ist, die den zylindrischen Innenraum (2) der Kartusche (1) mit der äußeren Umgebung verbindet, wobei die mindestens eine durchgehende Verbindung (41) reversibel verschlossen oder verschließbar ist und wobei der reversible Verschluss der mindestens einen durchgehenden Verbindung (41) durch einen Dichtungskörper (42) erfolgt, der durch eine Schraube (550) oder eine Schraubkappe (350) gegen die mindestens eine durchgehende Verbindung gepresst wird, wobei die Schraube (550) oder die Schraubkappe (350) manuell von außen verdrehbar ist.

14. Vorrichtung nach einem der Ansprüche 9 oder 10, **dadurch gekennzeichnet, dass**
die Druckentlastungsvorrichtung (16, 116, 216, 316, 416, 516) einen Dorn (242) zum Durchstechen einer Wandung (241) der Kartusche (1) im Bereich des zweiten Hohlraums (14) aufweist, wobei der Dorn (242) beweglich gegen die Kartusche (1) gelagert ist, wobei der Dorn (242) ein Hohldorn mit einer Kanüle ist, die mit einem Reservoir zur Aufnahme der Monomerflüssigkeit (7) verbunden ist, oder der Dorn (242) nach dem Durchstechen der Wandung der Kartusche (1) manuell oder durch eine Feder (248) zurückziehbar ist, so dass er einen mit dem Dorn (242) gestochenen Durchgang zur Ableitung der Monomerflüssigkeit (7) aus dem zweiten Hohlraum (14) freilegt, wobei bevorzugt die Wandung (241) im Bereich des Dorns (242) dünner ist als im Rest der Kartusche (1).

15. Vorrichtung nach einem der Ansprüche 9 bis 14, **dadurch gekennzeichnet, dass**
der Antriebskolben (5) mit zumindest einem Dichtungsring (28, 30) gegen die Innenwand Kartusche (1) abgedichtet ist, bevorzugt mit zwei Dichtungsringen (28, 30) gegen die Innenwand Kartusche (1) abgedichtet ist, wobei die zwei Dichtungsringe (28, 30) in einer Richtung parallel zur Zylinderachse des zylindrischen Innenraums (2) voneinander beabstandet sind, besonders bevorzugt zumindest 5 mm voneinander beabstandet sind, wobei vorzugsweise
in einer den zweiten Hohlraum (14) begrenzenden Innenwand der Kartusche (1) zumindest eine Nut (441) angeordnet ist, wobei die zumindest eine Nut (441) parallel zur Zylinderachse des zylindrischen Innenraums (2) zumindest genauso lang ist wie der Durchmesser eines am dichtesten in Richtung des Kartuschenkopfs (3) angeordneten vorderen Dichtungsrings (28) des zumindest einen Dichtungsrings (28, 30), bevorzugt mindestens doppelt so lang ist wie der vordere Dichtungsring (28), oder wobei die zumindest eine Nut (441) parallel zur Zylinderachse des zylindrischen Innenraums (2) zumindest halb so lang ist wie der vordere Dichtungsring (28) und entlang des Zylindermantels zumindest 4 mm breit ist, vorzugsweise zumindest 8 mm breit ist.

## Claims

1. A method for producing a bone cement, in particular a pasty polymethyl methacrylate bone cement, wherein the bone cement is produced from a cement powder (6) and a monomer liquid (7) using a device for mixing the bone cement, the device comprising
a cartridge (1) having a cylindrical interior (2),
a cement powder (6) for producing the bone cement,
a monomer liquid (7) for producing the bone cement, wherein the monomer liquid (7) is contained in a monomer liquid container (8),
a cartridge head (3) having a discharge opening (9) for expelling the bone cement, wherein the cartridge head (3) closes off the cylindrical interior (2) of the cartridge (1) at a front side of the cartridge (1), except for the discharge opening (9),
a closure (10, 210), wherein the closure (10, 210) is permeable to gases and impermeable to powder particles of the cement powder (6), and wherein the closure (10, 210) is arranged in the discharge opening (9) and is removable from the discharge opening (9), a drive piston (5), wherein the drive piston (5) is impermeable to gases and the monomer liquid (7), wherein the drive piston (5) is arranged so as to move toward the cartridge head (3) in the cylindrical interior (2) of the cartridge (1),
a central piston (4), wherein the central piston (4) is permeable to gases and the monomer liquid (7) and impermeable to the powder particles of the cement powder (6), wherein the central piston (4) is arranged so as to move in the cylindrical interior (2) of the cartridge (1) toward the cartridge head (3) and is arranged between the drive piston (5) and the cartridge head (3), wherein the central piston (4) separates the cylindrical interior (2) of the cartridge (1) into a first cavity (12) and a second cavity (14), wherein the first cavity (12) is delimited on a front side by the cartridge head (3) and the closure (10, 210), opposite thereto by the central piston (4), and laterally by an inner wall of the cartridge (1), and wherein the second cavity (14) is delimited on a front side by the central piston (4), opposite thereto by the drive piston (5), and laterally by the inner wall of the cartridge (1), wherein the cement powder (6) is arranged in the first cavity (12), and wherein the monomer liquid container (8) is arranged in the second cavity (14), wherein the method comprises the following steps:
A) moving the drive piston (5) toward the cartridge head (3),
B) opening the monomer liquid container (8) or rupturing the monomer liquid container (8) via the movement of the drive piston (5) toward the cartridge head (3), and thereby releasing the monomer liquid (7) in the second cavity (14),
C) expelling residual gases through the central piston (4), through the cement powder (6), and through the closure (10, 210), into the surroundings of the device, by means of the movement of the drive piston (5),
D) injecting the monomer liquid (7) through the central piston (4) into the cement powder (6), via further movement of the drive piston (5) toward the cartridge head (3),
E) displacing gases between the powder particles with the inflowing monomer liquid (7), wherein the gases escape through the closure (10, 210) into the surroundings of the device,
F) wetting the powder particles of the cement powder (6), **characterized by** the further method step
G) depressurizing the second cavity (14) after steps A) to F), wherein the second cavity (14) is depressurized by partially removing the monomer liquid (7) contained in the second cavity (14).

2. The method according to claim 1, **characterized in that**
the pressure in the second cavity (14) is reduced by at least 30% by the depressurization, particularly preferably is reduced by at least 60%, most particularly preferably is reduced by at least 90%.

3. The method according to claim 1 or 2, **characterized in that**
the movement of the drive piston (5) toward the cartridge head (3) is driven by an external extrusion device, wherein the device for mixing the bone cement is preferably inserted into the extrusion device and/or is attached to the extrusion device.

4. The method according to any one of the preceding claims, **characterized in that**, in step F), the powder particles of the cement powder (6) are completely wetted by the monomer liquid (7).

5. The method according to any one of the preceding claims, **characterized by** the chronological steps of
H) removing the closure (10, 210) from the cartridge head (3) after step G), and
I) extruding the bone cement out of the first cavity (12) and through the discharge opening (9) via a movement of the central piston (4) toward the cartridge head (3), wherein the central piston (4) is pressed by the drive piston (5) toward the cartridge head (3).

6. The method according to any one of the preceding claims, **characterized in that** the method is not used for the medical, diagnostic, or therapeutic treatment of a human or animal body.

7. The method according to any one of the preceding claims, **characterized in that**
the depressurization in step G) takes place via manual or automatic actuation of a depressurization device (16, 116, 216, 316, 416, 516) of the device for mixing the bone cement, wherein preferably the automatic actuation of the depressurization device (16, 116, 216, 316, 416, 516) is triggered by a movement of the drive piston (5), and/or is triggered by a movement of the closure (10, 210) out of the cartridge head (3), and is particularly preferably driven by the movement of the closure (10, 210) out of the cartridge head (3), and/or
during the depressurization in step G), the monomer liquid (7) is discharged into a reservoir outside the cartridge (1) or in the cartridge wall, wherein the reservoir is preferably sealed tightly to the outside for the monomer liquid (7), or
during the depressurization in step G), the monomer liquid (7) is conducted into a reservoir within the drive piston (5), into a reservoir on the side of the drive piston (5) opposite the second cavity (14), and/or into a reservoir laterally between the drive piston (5) and the cartridge (1), wherein, preferably in the latter case, the monomer liquid (7) is conducted via at least one groove (441) in an inner wall of the cartridge (1), past a front sealing ring (28) of the drive piston (5), into the reservoir laterally between the drive piston (5) and the cartridge (1), when a front sealing ring (28) of the drive piston (5) is pushed over or onto the at least one groove (441).

8. The method according to any one of the preceding claims, **characterized in that**
during the depressurization in step G), the monomer liquid (7) is not conducted into the first cavity (12), is preferably conducted into at least one reservoir separate from the first cavity (12), wherein particularly preferably the at least one reservoir is arranged outside the cartridge (1), in the cartridge wall, within the drive piston (5), on the side of the drive piston (5) opposite the second cavity (14), and/or laterally between the drive piston (5) and the cartridge (1), and/or
during depressurization in step G), the second cavity (14) is filled with the open or ruptured monomer liquid container (8) and with residues of the monomer liquid (7).

9. A device for mixing a bone cement, the device comprising
a cartridge (1) having a cylindrical interior (2),
a cement powder (6) for producing the bone cement,
a monomer liquid (7) for producing the bone cement, wherein the monomer liquid (7) is contained in a monomer liquid container (8),
a cartridge head (3) having a discharge opening (9) for expelling the bone cement, wherein the cartridge head (3) closes off the cylindrical interior (2) of the cartridge (1) at a front side of the cartridge (1), except for the discharge opening (9),
a closure (10, 210), wherein the closure (10, 210) is permeable to gases and impermeable to powder particles of the cement powder (6), and wherein the closure (10, 210) is arranged in the discharge opening (9) and is removable from the discharge opening (9), a drive piston (5), wherein the drive piston (5) is impermeable to gases and the monomer liquid (7), wherein the drive piston (5) is arranged so as to move toward the cartridge head (3) in the cylindrical interior (2) of the cartridge (1),
a central piston (4), wherein the central piston (4) is permeable to gases and the monomer liquid (7) and impermeable to the powder particles of the cement powder (6), wherein the central piston (4) is arranged so as to move in the cylindrical interior (2) of the cartridge (1) toward the cartridge head (3) and is arranged between the drive piston (5) and the cartridge head (3), wherein the central piston (4) separates the cylindrical interior (2) of the cartridge (1) into a first cavity (12) and a second cavity (14), wherein the first cavity (12) is delimited on a front side by the cartridge head (3) and the closure (10, 210), opposite thereto by the central piston (4) and laterally by an inner wall of the cartridge (1), and wherein the second cavity (14) is delimited on a front side by the central piston (4), opposite thereto by the drive piston (5), and laterally by the inner wall of the cartridge (1), wherein the cement powder (6) is arranged in the first cavity (12) and wherein the monomer liquid container (8) is arranged in the second cavity (14), **characterized in that** the device further comprises:
a depressurization device (16, 116, 216, 316, 416, 516) by means of which monomer liquid (7) can be discharged from the second cavity (14) or be discharged and received, or by means of which the volume of the second cavity (14) can be increased, wherein the depressurization device (16, 116, 216, 316, 416, 516) is connected or connectible to the second cavity (14).

10. The device according to claim 9, **characterized in that**
the device is suitable for implementing a method according to any one of claims 1 to 8.

11. The device according to claim 9 or 10, **characterized in that**
the depressurization device (16, 116, 216, 316, 416, 516) is connected to the second cavity (14) via at least one continuous connection (41) in a wall of the cartridge (1), wherein the at least one continuous connection (41) is closed or can be closed with at least one sealing body (42), wherein the at least one sealing body (42) is preferably pressed against the at least one continuous connection (41) with a screw (550) or screw cap (350) that can be manually operated from the outside, with a rod (346), or with a spring guide rod (46, 146, 246).

12. The device according to claim 11, **characterized in that**
the at least one sealing body (42), or a rod (346) or a spring guide rod (46, 146, 246) pressing onto the at least one sealing body (42), can be lifted from the at least one continuous connection (41) with a spring (48, 148, 248); preferably, the spring (48, 148, 248) presses the spring guide rod (46, 146, 246), which presses the at least one sealing body (42) against the at least one continuous connection (41), away from the at least one continuous connection (41), wherein the spring (48, 148) is particularly preferably locked with a pin (40) or with a fork-shaped supporting body (140), and the pin (40) or the fork-shaped supporting body (140) is firmly connected to the closure (10) such that, when the closure (10) moves out of the discharge opening (9), the pin (40) or the fork-shaped supporting body (140) is also automatically removed from the depressurization device (16, 116), and the locking of the spring (48, 148) is thereby released.

13. The device according to claim 9 or 10, **characterized in that**
at least one continuous connection (41) is arranged in the cartridge wall and connects the cylindrical interior (2) of the cartridge (1) to the external surroundings, wherein the at least one continuous connection (41) is reversibly closed or closable, and wherein the reversible closure of the at least one continuous connection (41) takes place via a sealing body (42) which is pressed against the at least one continuous connection by a screw (550) or a screw cap (350), wherein the screw (550) or the screw cap (350) is manually rotatable from the outside.

14. The device according to any one of claims 9 or 10, **characterized in that**
the depressurization device (16, 116, 216, 316, 416, 516) has a spike (242) for piercing a wall (241) of the cartridge (1) in the region of the second cavity (14), wherein the spike (242) is movably mounted against the cartridge (1), wherein the spike (242) is a hollow spike with a cannula that is connected to a reservoir for receiving the monomer liquid (7), or, after piercing the wall of the cartridge (1), the spike (242) can be retracted manually or by a spring (248) such that it exposes a passage pierced with the spike (242) to discharge the monomer liquid (7) from the second cavity (14), wherein the wall (241) is preferably thinner in the region of the spike (242) than in the rest of the cartridge (1).

15. The device according to any one of claims 9 to 14, **characterized in that**
the drive piston (5) is sealed with at least one sealing ring (28, 30) against the inner wall cartridge (1), is preferably sealed with two sealing rings (28, 30) against the inner wall cartridge (1), wherein the two sealing rings (28, 30) are spaced apart from one another, particularly preferably spaced apart from one another by at least 5 mm, in a direction parallel to the cylinder axis of the cylindrical interior (2), wherein preferably at least one groove (441) is arranged in an inner wall of the cartridge (1) which delimits the second cavity (14), wherein the at least one groove (441) parallel to the cylinder axis of the cylindrical interior (2) is at least as long as the diameter of a front sealing ring (28) of the at least one sealing ring (28, 30) arranged closest toward the cartridge head (3), preferably at least twice as long as the front sealing ring (28), or wherein the at least one groove (441) parallel to the cylinder axis of the cylindrical interior (2) is at least half as long as the front sealing ring (28) and is at least 4 mm wide, preferably at least 8 mm wide, along the cylinder shell.

## Revendications

1. Procédé de fabrication d'un ciment osseux, en particulier d'un ciment osseux à base de polyméthacrylate de méthyle sous forme de pâte, dans lequel le ciment osseux est fabriqué à partir d'une poudre de ciment (6) et d'un fluide monomère (7) avec un dispositif pour le mélange du ciment osseux, le dispositif présentant
une cartouche (1) comportant un espace interne cylindrique (2),
une poudre de ciment (6) pour la fabrication du ciment osseux,
un fluide monomère (7) pour la fabrication du ciment osseux, dans lequel le fluide monomère (7) est contenu dans un récipient de fluide monomère (8),
une tête de cartouche (3) comportant une ouverture de décharge (9) pour l'éjection du ciment osseux, dans lequel la tête de cartouche (3) ferme l'espace interne cylindrique (2) de la cartouche (1) au niveau d'une face avant de la cartouche (1) jusqu'à l'ouverture de décharge (9),
une fermeture (10, 210), dans lequel la fermeture (10, 210) est perméable aux gaz et imperméable aux particules de poudre de la poudre de ciment (6) et dans lequel la fermeture (10, 210) est disposée dans l'ouverture de décharge (9) et peut être retirée de l'ouverture de décharge (9),
un piston d'entraînement (5), dans lequel le piston d'entraînement (5) est imperméable aux gaz et au fluide monomère (7), dans lequel le piston d'entraînement (5) est disposé mobile en direction de la tête de cartouche (3) dans l'espace interne cylindrique (2) de la cartouche (1), un piston central (4), dans lequel le piston central (4) est perméable aux gaz et au fluide monomère (7) et imperméable aux particules de poudre de la poudre de ciment (6), dans lequel le piston central (4) est disposé mobile en direction de la tête de cartouche (3) dans l'espace interne cylindrique (2) de la cartouche (1) et est disposé entre le piston d'entraînement (5) et la tête de cartouche (3), dans lequel le piston central (4) sépare l'espace interne cylindrique (2) de la cartouche (1) en une première cavité (12) et en une seconde cavité (14), dans lequel la première cavité (12) est délimitée au niveau d'une face avant par la tête de cartouche (3) et la fermeture (10, 210), à l'opposé par le piston central (4) et latéralement par une paroi interne de la cartouche (1) et dans lequel la seconde cavité (14) est délimitée au niveau d'une face avant par le piston central (4), à l'opposé par le piston d'entraînement (5) et latéralement par la paroi interne de la cartouche (1), dans lequel la poudre de ciment (6) est disposée dans la première cavité (12) et dans lequel le récipient de fluide monomère (8) est disposé dans la seconde cavité (14), dans lequel le procédé présente les étapes suivantes :
A) déplacement du piston d'entraînement (5) en direction de la tête de cartouche (3),
B) ouverture du récipient de fluide monomère (8) ou rupture du récipient de fluide monomère (8) par le déplacement du piston d'entraînement (5) en direction de la tête de cartouche (3) et ainsi libération du fluide monomère (7) dans la seconde cavité (14),
C) expulsion par pression des gaz en excès à travers le piston central (4), à travers la poudre de ciment (6) et à travers la fermeture (10, 210) dans l'environnement du dispositif au moyen du déplacement du piston d'entraînement (5),
D) poussée par pression du fluide monomère (7) à travers le piston central (4) dans la poudre de ciment (6) par le biais d'un déplacement supplémentaire du piston d'entraînement (5) en direction de la tête de cartouche (3),
E) refoulement des gaz entre les particules de poudre avec le fluide monomère (7) en écoulement, dans lequel les gaz s'échappent à travers la fermeture (10, 210) dans l'environnement du dispositif,
F) humidification des particules de poudre de la poudre de ciment (6),
**caractérisé par** l'étape de procédé supplémentaire de :
G) décompression de la seconde cavité (14) après les étapes A) à F), dans lequel la décompression de la seconde cavité (14) est effectuée par un retrait partiel du fluide monomère (7) contenu dans la seconde cavité (14).

2. Procédé selon la revendication 1, **caractérisé en ce que**
la pression dans la seconde cavité (14) est réduite par la décompression d'au moins 30 %, de manière particulièrement préférée est réduite d'au moins 60 %, de manière tout particulièrement préférée est réduite d'au moins 90 %.

3. Procédé selon la revendication 1 ou 2, **caractérisé en ce que**
le déplacement du piston d'entraînement (5) en direction de la tête de cartouche (3) est entraîné par un dispositif d'expulsion par pression externe, dans lequel de préférence le dispositif pour le mélange du ciment osseux est inséré dans le dispositif d'expulsion par pression et/ou est fixé au dispositif d'expulsion par pression.

4. Procédé selon l'une des revendications précédentes, **caractérisé en ce que**, à l'étape F), les particules de poudre de la poudre de ciment (6) sont totalement humidifiées par le fluide monomère (7).

5. Procédé selon l'une des revendications précédentes, **caractérisé par** les étapes chronologiques de
H) retrait de la fermeture (10, 210) de la tête de cartouche (3) après l'étape G) et
I) expulsion par pression du ciment osseux hors de la première cavité (12) et à travers l'ouverture de décharge (9) par le biais d'un déplacement du piston central (4) en direction de la tête de cartouche (3), dans lequel le piston central (4) est pressé par le piston d'entraînement (5) en direction de la tête de cartouche (3).

6. Procédé selon l'une des revendications précédentes, **caractérisé en ce que** le procédé n'est pas utilisé pour le traitement médical, diagnostique ou thérapeutique d'un corps humain ou animal.

7. Procédé selon l'une des revendications précédentes, **caractérisé en ce que** la décompression à l'étape G) est effectuée par un actionnement manuel ou automatique d'un dispositif de décompression (16, 116, 216, 316, 416, 516) du dispositif pour le mélange du ciment osseux, dans lequel de préférence l'actionnement automatique du dispositif de décompression (16, 116, 216, 316, 416, 516) est déclenché par un déplacement du piston d'entraînement (5) et/ou est déclenché par un déplacement de la fermeture (10, 210) hors de la tête de cartouche (3) et de manière particulièrement préférée est entraîné par le déplacement de la fermeture (10, 210) hors de la tête de cartouche (3), et/ou lors de la décompression à l'étape G), le fluide monomère (7) est dérivé dans un réservoir à l'extérieur de la cartouche (1) ou dans la paroi de cartouche, dans lequel de préférence le réservoir est fermé de manière étanche vers l'extérieur pour le fluide monomère (7), ou lors de la décompression à l'étape G), le fluide monomère (7) est dirigé dans un réservoir à l'intérieur du piston d'entraînement (5), dans un réservoir sur la face du piston d'entraînement (5) opposée à la seconde cavité (14) et/ou dans un réservoir latéralement entre le piston d'entraînement (5) et la cartouche (1), dans lequel de préférence, dans le dernier cas, le fluide monomère (7) est dirigé à travers au moins une rainure (441) dans une paroi interne de la cartouche (1), le long d'une bague d'étanchéité avant (28) du piston d'entraînement (5), dans le réservoir latéralement entre le piston d'entraînement (5) et la cartouche (1), lorsqu'une bague d'étanchéité avant (28) du piston d'entraînement (5) est poussée au-dessus de l'au moins une rainure (441) ou sur celle-ci.

8. Procédé selon l'une des revendications précédentes, **caractérisé en ce que**, lors de la décompression à l'étape G), le fluide monomère (7) n'est pas dirigé dans la première cavité (12), de préférence est dirigé dans au moins un réservoir séparé de la première cavité (12), dans lequel, de manière particulièrement préférée, l'au moins un réservoir est disposé à l'extérieur de la cartouche (1), dans la paroi de cartouche, à l'intérieur du piston d'entraînement (5), sur la face du piston d'entraînement (5) opposée à la seconde cavité (14) et/ou latéralement entre le piston d'entraînement (5) et la cartouche (1), et/ou lors de la décompression à l'étape G), la seconde cavité (14) est remplie avec le récipient de fluide monomère (8) ouvert ou rompu et avec le reste du fluide monomère (7).

9. Dispositif pour le mélange d'un ciment osseux, le dispositif présentant
une cartouche (1) comportant un espace interne cylindrique (2),
une poudre de ciment (6) pour la fabrication du ciment osseux,
un fluide monomère (7) pour la fabrication du ciment osseux, dans lequel le fluide monomère (7) est contenu dans un récipient de fluide monomère (8),
une tête de cartouche (3) comportant une ouverture de décharge (9) pour l'éjection du ciment osseux, dans lequel la tête de cartouche (3) ferme l'espace interne cylindrique (2) de la cartouche (1) au niveau d'une face avant de la cartouche (1) jusqu'à l'ouverture de décharge (9),
une fermeture (10, 210), dans lequel la fermeture (10, 210) est perméable aux gaz et imperméable aux particules de poudre de la poudre de ciment (6) et dans lequel la fermeture (10, 210) est disposée dans l'ouverture de décharge (9) et peut être retirée de l'ouverture de décharge (9),
un piston d'entraînement (5), dans lequel le piston d'entraînement (5) est imperméable aux gaz et au fluide monomère (7), dans lequel le piston d'entraînement (5) est disposé mobile en direction de la tête de cartouche (3) dans l'espace interne cylindrique (2) de la cartouche (1), un piston central (4), dans lequel le piston central (4) est perméable aux gaz et au fluide monomère (7) et imperméable aux particules de poudre de la poudre de ciment (6), dans lequel le piston central (4) est disposé mobile en direction de la tête de cartouche (3) dans l'espace interne cylindrique (2) de la cartouche (1) et est disposé entre le piston d'entraînement (5) et la tête de cartouche (3), dans lequel le piston central (4) sépare l'espace interne cylindrique (2) de la cartouche (1) en une première cavité (12) et en une seconde cavité (14), dans lequel la première cavité (12) est délimitée au niveau d'une face avant par la tête de cartouche (3) et la fermeture (10, 210), à l'opposé par le piston central (4) et latéralement par une paroi interne de la cartouche (1) et dans lequel la seconde cavité (14) est délimitée au niveau d'une face avant par le piston central (4), à l'opposé par le piston d'entraînement (5) et latéralement par la paroi interne de la cartouche (1), dans lequel la poudre de ciment (6) est disposée dans la première cavité (12) et dans lequel le récipient de fluide monomère (8) est disposé dans la seconde cavité (14), **caractérisé en ce que** le dispositif présente en outre :
un dispositif de décompression (16, 116, 216, 316, 416, 516), avec lequel le fluide monomère (7) peut être dérivé depuis la seconde cavité (14) ou peut être dérivé depuis celle-ci et extrait de celle-ci ou avec lequel le volume de la seconde cavité (14) peut être augmenté, dans lequel le dispositif de décompression (16, 116, 216, 316, 416, 516) est relié ou peut être relié à la seconde cavité (14).

10. Dispositif selon la revendication 9, **caractérisé en ce que**
le dispositif est adapté pour mettre en oeuvre un procédé selon l'une des revendications 1 à 8.

11. Dispositif selon la revendication 9 ou 10, **caractérisé en ce que**
le dispositif de décompression (16, 116, 216, 316, 416, 516) est relié à la seconde cavité (14) par l'intermédiaire d'au moins une liaison continue (41) dans une paroi de la cartouche (1), dans lequel l'au moins une liaison continue (41) est fermée ou peut être fermée avec au moins un corps d'étanchéité (42), dans lequel de préférence l'au moins un corps d'étanchéité (42) est pressé contre l'au moins une liaison continue (41) avec une vis (550) ou un bouchon à vis (350) actionnable manuellement depuis l'extérieur, avec une tige (346) ou avec une tige de guidage de ressort (46, 146, 246).

12. Dispositif selon la revendication 11, **caractérisé en ce que**
l'au moins un corps d'étanchéité (42) ou une tige (346) ou tige de guidage de ressort (46, 146, 246) pressant sur l'au moins un corps d'étanchéité (42) peut être détaché de l'au moins une liaison continue (41) avec un ressort (48, 148, 248), de préférence le ressort (48, 148, 248) presse la tige de guidage de ressort 46, 146, 246), laquelle presse l'au moins un corps d'étanchéité (42) contre l'au moins une liaison continue (41), de manière à l'éloigner de l'au moins une liaison continue (41), dans lequel, de manière particulièrement préférée, le ressort (48, 148) est bloqué avec un tenon (40) ou avec un corps d'appui en forme de fourche (140) et le tenon (40) ou le corps d'appui en forme de fourche (140) est relié de manière fixe à la fermeture (10), de sorte que, lors d'un déplacement de la fermeture (10) hors de l'ouverture de décharge (9), le tenon (40) ou le corps d'appui en forme de fourche (140) est également automatiquement retiré du dispositif de décompression (16, 116) et ainsi le blocage du ressort (48, 148) est libéré.

13. Dispositif selon la revendication 9 ou 10, **caractérisé en ce que**
au moins une liaison continue (41) est disposée dans la paroi de cartouche, laquelle liaison continue relie l'espace interne cylindrique (2) de la cartouche (1) à l'environnement extérieur, dans lequel l'au moins une liaison continue (41) est fermée ou peut être fermée de manière réversible et dans lequel la fermeture réversible de l'au moins une liaison continue (41) est effectuée par un corps d'étanchéité (42) qui est pressé par une vis (550) ou un bouchon à vis (350) contre l'au moins une liaison continue, dans lequel la vis (550) ou le bouchon à vis (350) peut être entraîné en rotation manuellement depuis l'extérieur.

14. Dispositif selon l'une des revendications 9 ou 10, **caractérisé en ce que**
le dispositif de décompression (16, 116, 216, 316, 416, 516) présente un mandrin (242) permettant de percer une paroi (241) de la cartouche (1) dans la zone de la seconde cavité (14), dans lequel le mandrin (242) est monté mobile contre la cartouche (1), dans lequel le mandrin (242) est un mandrin creux comportant une canule qui est reliée à un réservoir pour la réception du fluide monomère (7), ou le mandrin (242) peut être rétracté manuellement ou par un ressort (248) après le perçage de la paroi de la cartouche (1), de sorte qu'il libère un passage percé avec le mandrin (242) pour la dérivation du fluide monomère (7) hors de la seconde cavité (14), dans lequel de préférence la paroi (241) est plus fine dans la zone du mandrin (242) que dans le reste de la cartouche (1).

15. Dispositif selon l'une des revendications 9 à 14, **caractérisé en ce que**
le piston d'entraînement (5) est scellé avec au moins une bague d'étanchéité (28, 30) contre la paroi interne cartouche (1), de préférence est scellé avec deux bagues d'étanchéité (28, 30) contre la paroi interne cartouche (1), dans lequel les deux bagues d'étanchéité (28, 30) sont espacées l'une de l'autre dans une direction parallèle à l'axe de cylindre de l'espace interne cylindrique (2), de manière particulièrement préférée sont espacées l'une de l'autre d'au moins 5 mm, dans lequel de préférence au moins une rainure (441) est disposée dans une paroi interne de la cartouche (1) délimitant la seconde cavité (14), dans lequel l'au moins une rainure (441), parallèlement à l'axe de cylindre de l'espace interne cylindrique (2), est au moins aussi longue que le diamètre d'une bague d'étanchéité avant (28), disposée la plus étanche en direction de la tête de cartouche (3), de l'au moins une bague d'étanchéité (28, 30), de préférence est au moins deux fois plus longue que la bague d'étanchéité avant (28), ou dans lequel l'au moins une rainure (441), parallèlement à l'axe de cylindre de l'espace interne cylindrique (2), est au moins moitié moins longue que la bague d'étanchéité avant (28) et est d'une largeur d'au moins 4 mm le long de l'enveloppe de cylindre, de préférence est d'une largeur d'au moins 8 mm.
